# EUROPÄISCHE PATENTSCHRIFT

(11) **EP 2 194 096 B1**
(45) Veröffentlichungstag und Bekanntmachung des Hinweises auf die Patenterteilung: **11.06.2014**
(21) Anmeldenummer: 10000387.0
(22) Anmeldetag: 28.11.2001
(51) Int. Cl.: C08L 51/00, C08F 290/06, C08F 291/00, C08F 2/22, C08F 265/04, C08F 271/02

(54) **Verwendung von kammförmigen Copolymeren auf Basis von Acryloyldimethyltaurinsäure in kosmetischen, pharmazeutischen und dermatologischen Mitteln**
Use of pectinate copolymers on the basis of acryloyldimethyl taurine acid in cosmetic, dermatological and pharmaceutical agents
Utilisation de copolymères en forme de peigne à base d'acide d'acryloyldiméthyle-taurine dans des agents cosmétiques, pharmaceutiques et dermatologiques

(30) Priorität: 01.12.2000 DE 10059825
(43) Veröffentlichungstag der Anmeldung: 09.06.2010
(62) Teilanmeldung aus: 01989526.7
(73) Patentinhaber: Clariant Produkte (Deutschland) GmbH, 65929 Frankfurt am Main (DE)
(72) Erfinder: Loeffler, Matthias, 65510 Idstein (DE); Morschhaeuser, Roman, 55122 Mainz (DE); Schade, Manfred, 68200 Mulhouse (FR)
(74) Vertreter: Paczkowski, Marcus

(56) Entgegenhaltungen:
- EP-A1- 0 815 828
- EP-A1- 0 815 844
- EP-A1- 0 815 845
- EP-A1- 0 829 258
- EP-A1- 0 919 217
- EP-A2- 0 356 241
- US-A- 4 521 578
- US-A- 5 368 850

## Beschreibung

Die vorliegende Erfindung betrifft die Verwendung von Copolymeren auf Basis von Acryloyldimethyltaurinsäure und Makromonomeren gemäß Formel (IV) als Verdicker, Dispergiermittel, Suspendiermittel, Emulgator, Stabilisator, Solubilisator, Konditionierer, Konsistenzgeber, Gleitmittel, Haftmittel und/oder Conditioner in kosmetischen, pharmazeutischen und dermatologischen Mitteln.

Verbraucherwünsche und Rheologie kosmetischer Produkte sind eng miteinander verknüpft. So wird. z.B. das visuelle Erscheinungsbild einer Creme oder Lotion durch die Viskosität beeinflusst. Die sensorischen Eigenschaften, wie Konsistenz oder Verteilbarkeit bestimmen das individuelle Profil eines Kosmetikproduktes. Auch die Effektivität von Wirksubstanzen (z.B. Sonnenschutzfilter) und auch die Lagerstabilität der Formulierung steht in engem Zusammenhang mit den rheologischen Eigenschaften der Produkte.

Im kosmetischen Bereich kommt Polyelektrolyten als Verdicker und Gelbildner eine tragende Rolle zu. Stand der Technik sind insbesondere Verdicker auf Basis der Poly(meth)acrylsäure und deren wasserlöslichen Copolymeren. Die Vielfalt der möglichen Strukturen und die damit verbundenen vielfältigen Anwendungsmöglichkeiten drücken sich nicht zuletzt in einer Vielzahl von Patenten aus, die seit Mitte der 70iger Jahre weltweit angemeldet wurden.
Ein wesentlicher Nachteil der Verdicker auf Basis von Poly(meth)acrylsäure ist die starke pH-Abhängigkeit der Verdickungsleistung. So wird im allgemeinen ein hinreichend hohe Viskosität nur dann aufgebaut, wenn der pH Wert der Formulierung oberhalb von pH 6 eingestellt, d.h. die Poly(meth)acrylsäure in neutralisierter Form vorliegt. Ferner sind die entsprechenden Mittel empfindlich gegenüber UV-Strahlung und auch Scherung und vermitteln zudem auf der Haut zudem ein klebriges Gefühl. Ebenso ist die Handhabung derartiger Verdicker problematisch. Da die Verdicker i.a. in saurer Form vorliegen, bedarf es bei der Formulierung eines zusätzlichen Neutralisationsschrittes. In den 90iger Jahren wurden neuartige Verdicker auf Basis vernetzter und neutralisierter Polyacryloyldimethyltaurate in den Markt eingeführt (EP-B-0 815 828, EP-B-0 815 844, EP-B-0 815 845 EP-A-0829258, EP-A-0919217, US 5368850, EP 0356241, und EP-A-0 850 642). Sowohl in Form des vorneutralisierten Homopolymers als auch als korrespondierendes Copolymer (^{®}ARISTOFLEX AVC, CLARIANT GmbH) sind diese Verdicker den Poly(meth)acrylat-Typen in vieler Hinsicht überlegen. Beispielsweise zeigen Acryloyldimethyltaurat-basierende Verdickersysteme hervorragende Eigenschaften in pH-Bereichen unterhalb von pH 6, also in einem pH-Bereich, in dem mit herkömmlichen Poly(meth)acrylat-Verdickern nicht mehr gearbeitet werden kann. Hohe UV- und Scherstabilität, hervorragende viskoelastische Eigenschaften, leichte Verarbeitbarkeit und ein günstiges toxikologisches Profil des Hauptmonomeren machen Acryloyldimethyltaurat-basierende Verdickersysteme zu modernen, neuen Vertretern mit hohem Potenzial für die Zukunft.

Im Laufe der letzten Jahre etablierte sich ein weiteres Verdickerkonzept auf dem Markt. Hierbei wurde durch hydrophobe Modifikation der konventionellen Poly(meth)acrylate der Zugang zu Polymeren gefunden, die sowohl verdickende als auch emulgierende/dispergierende Eigenschaften aufweisen. Beispiele für kommerzielle hydrophob modifizierte Poly(meth)acrylate sind ^{®}PEMULEN TR-1 und TR-2 von BF-Goodrich und ^{®}ACULYN 22 Rohm und Haas. Da diese hydrophob modifizierte Polymere auf der Basis von (Meth)acrylsäure aufgebaut sind, besitzen sie auch die oben erwähnten Nachteile der Poly(meth)acrylate.

Überraschend wurde nun gefunden, dass sich eine neue Klasse von Kammpolymeren auf Basis von Acryloyldimethyltaurinsäure (AMPS) - die sich sowohl als Verdicker, Konsistenzgeber, Emulgator, Solubilisator, Dispergator, Gleitmittel, Haftmittel, Conditioner und/oder Stabilisator eignen - hervorragend zur Formulierung von kosmetischen, pharmazeutischen und dermatologischen Mitteln geeignet sind.

Gegenstand der Erfindung ist daher die Verwendung von Copolymeren, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt, und die Makromonomere acrylisch- oder methacrylisch monofunktionalisierte Alkylethoxylate gemäß Formel (IV) sind 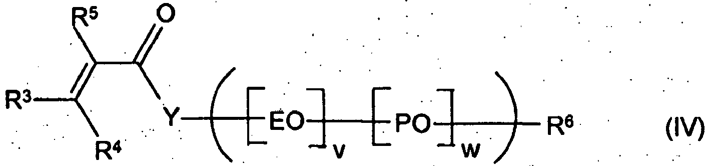 worin
   R3 und R₄ gleich H oder -CH₃ sind, R₅ gleich H oder -CH₃ ist; und R₆ gleich einem n-aliphatischen, iso-aliphatischen, olefinischen, cycloaliphatischen, arylaliphatischen oder aromatischen (C₁-C₃₀)-Kohlenwasserstoffrest ist, v und w die stöchiometrischen Koeffizienten betreffend die Ethylenoxideinheiten (EO) und Propylenoxideinheiten (PO) sind, wobei v und w unabhängig voneinander 0 bis 500 betragen, die Summe aus v und w im Mittel ≥ 1 sein muss und die Verteilung der EO- und PO-Einheiten über die Makromonomerkette statistisch, blockartig, alternierend oder gradientenartig sein kann und Y -O-, -C(O)-, -C(O)-O-, -S-, -O-CH₂-CH(O-)-CH₂OH, -O-CH₂CH(OH)-CH₂O-, -O-SO₂-O-, -O-SO-O-, -PH-, -P(CH₃)-, -PO₃-, -NH- oder -N(CH₃)- ist,
G) wobei die Copolymerisation gegebenenfalls in Gegenwart mindestens eines polymeren Additivs mit zahlenmittleren Molekulargewichten von 200 g/mol bis 10⁹ g/mol erfolgt,
als Verdicker, Dispergiermittel, Suspendiermittel, Emulgator, Stabilisator, Solubilisator, Konditionierer, Konsistenzgeber, Gleitmittel, Haftmittel und/oder Conditioner in kosmetischen, pharmazeutischen und dermatologischen Mitteln.

Die erfindungsgemäßen Copolymere besitzen bevorzugt ein Molekulargewicht von 10³ g/mol bis 10⁹ g/mol, besonders bevorzugt von 10⁴ bis 10⁷ g/mol, insbesondere bevorzugt 5*10⁴ bis 5*10⁶ g/mol.

Bei den Acryloyldimethyltauraten kann es sich um die anorganischen oder organischen Salze der Acryloyldimethyltaurinsäure handeln. Bevorzugt werden die Li⁺-, Na⁺-, K⁺-, Mg⁺⁺-, Ca⁺⁺-, Al⁺⁺⁺- und/oder NH₄⁺-Salze. Ebenfalls bevorzugt sind die Monoalkylammonium-, Dialkylammonium-, Trialkylammonium- und/oder Tetraalkylammoniumsalze, wobei es sich bei den Alkylsubstituenten der Amine unabhängig voneinander um (C₁-C₂₂)-Alkylreste oder (C₂-C₁₀)-Hydroxyalkylreste handeln kann. Weiterhin sind auch ein bis dreifach ethoxylierte Ammoniumverbindungen mit unterschiedlichem Ethoxylierungsgrad bevorzugt. Es sollte angemerkt werden, dass auch Mischungen von zwei- oder mehreren der oben genannten Vertreter im Sinne der Erfindung sind.

Der Neutralisationsgrad der Acryloyldimethyltaurinsäure kann zwischen 0 und 100 % betragen, besonders bevorzugt ist ein Neutralisationsgrad von oberhalb 80 %.

Bezogen auf die Gesamtmasse der Copolymere beträgt der Gehalt an Acryloyldimethyltaurinsäure bzw. Acryloyldimethyltauraten mindestens 0,1 Gew.-%, bevorzugt 20 bis 99,5 Gew.-%, besonders bevorzugt 50 bis 98 Gew.-%.

Als Comonomere B) können alle olefinisch ungesättigten, nicht kationischen Monomere eingesetzt werden, deren Reaktionsparameter eine Copolymerisation mit Acryloyldimethyltaurinsäure und/oder Acryloyldimethyltauraten in den jeweiligen Reaktionsmedien erlauben.
Als Comonomere B) bevorzugt sind ungesättigte Carbonsäuren und deren Anhydride und Salze, sowie deren Ester mit aliphatischen, olefinischen, cycloaliphatischen, arylaliphatischen oder aromatischen Alkoholen mit einer Kohlenstoffzahl von 1 bis 30.

Als ungesättigte Carbonsäuren besonders bevorzugt sind Acrylsäure, Methacrylsäure, Styrolsulfonsäure, Maleinsäure, Fumarsäure, Crotonsäure, Itaconsäure und Seneciosäure.
Als Gegenionen bevorzugt sind Li⁺, Na⁺, K⁺, Mg⁺⁺, Ca⁺⁺, Al⁺⁺⁺, NH₄⁺, Monoalkylammonium-, Dialkylammonium-, Trialkylammonium- und/oder Tetraalkylammoniumreste, wobei es sich bei den Alkylsubstituenten der Amine unabhängig voneinander um (C₁-C₂₂)-Alkylreste oder (C₂-C₁₀)-Hydroxyalkylreste handeln kann. Zusätzlich können auch ein bis dreifach ethoxylierte Ammoniumverbindungen mit unterschiedlichem Ethoxylierungsgrad Anwendung finden. Der Neutralisationsgrad der Carbonsäuren kann zwischen 0 und 100% betragen.
Als Comonomere B) weiterhin bevorzugt sind offenkettige N-Vinylamide, bevorzugt N-Vinylformamid (VIFA), N-Vinylmethylformamid, N-Vinylmethylacetamid (VIMA) und N-Vinylacetamid; cyclische N-Vinylamide (N-Vinyllactame) mit einer Ringgröße von 3 bis 9, bevorzugt N-Vinylpyrrolidon (NVP) und N-Vinylcaprolactam; Amide der Acryl- und Methacrylsäure, bevorzugt Acrylamid, Methacrylamid, N,N-Dimethyl-acrylamid, N,N-Diethylacrylamid und N,N-Diisopropylacrylamid; alkoxylierte Acryl- und Methacrylamide, bevorzugt Hydroxyethylmethacrylat, Hydroxymethylmethacrylamid, Hydroxyethylmethacrylamid, Hydroxypropylmethacrylamid und Bernsteinsäuremono-[2-(methacryloyloxy)-ethylester];
N,N-Dimethylaminomethacrylat; Diethylamino-methylmethacrylat; Acryl- und Methacrylamidoglykolsäure; 2- und 4-Vinylpyridin; Vinylacetat; Methacrylsäureglycidylester; Styrol; Acrylnitril; Vinylchlorid; Stearylacrylat; Laurylmethacrylat; Vinylidenchlorid; und/oder Tetrafluorethylen.
Als Comonomere B) ebenfalls geeignet sind anorganische Säuren und deren Salze und Ester. Bevorzugte Säuren sind Vinylphosphonsäure, Vinylsulfonsäure, Allylphosphonsäure und Methallylsulfonsäure.

Der Gewichtsanteil der Comonomere B), bezogen auf die Gesamtmasse der Copolymere, kann 0 bis 99,8 Gew.-% betragen und beträgt bevorzugt 0,5 bis 80 Gew.-%, besonders bevorzugt 2 bis 50 Gew.-%.

Als Comonomere C) kommen alle olefinisch ungesättigten Monomere mit kationischer Ladung in Frage, die in der Lage sind, in den gewählten Reaktionsmedien mit Acryloyldimethyltaurinsäure oder deren Salze Copolymere zu bilden. Die dabei resultierende Verteilung der kationischen Ladungen über die Ketten hinweg kann statistisch, alternierend, block- oder gradientenartig sein. Es sei darauf hingewiesen werden, dass unter den kationischen Comonomeren C) auch solche zu verstehen sind, die die kationische Ladung in Form einer betainischen, zwitterionischen, oder amphomeren Struktur tragen. Comonomere C) im Sinne der Erfindung sind auch aminofunktionalisierte Precursor, die durch polymeranaloge Reaktionen in Ihre entsprechenden quaternären (z.B. Reaktion mit Dimethylsulfat, Methylchlorid), zwitterionischen (z.B. Reaktion mit Wasserstoffperoxid), betainischen (z.B. Reaktion mit Chloressigsäure), oder amphoteren Derivate überführt werden können.

Besonders bevorzugt als Comonomere C) sind
Diallyldimethylammoniumchlorid (DADMAC),
[2-(Methacryloyloxy)ethyl]trimethylammoniumchlorid (MAPTAC),
[2-(Acryloyloxy)ethyl]trimethylammoniumchlorid,
[2-Methacrylamidoethyl]trimethylammoniumchlorid,
[2-(Acrylamido)ethyl]trimethylammoniumchlorid,
N-Methyl-2-vinylpyridiniumchlorid
N-Methyl-4-vinylpyridiniumchlorid
Dimethylaminoethylmethacrylat,
Dimethylaminopropylmethacrylamid,
Methacryloylethyl-N-oxid und/oder
Methacryloylethyl-betain.

Der Gewichtsanteil der Comonomeren C), bezogen auf die Gesamtmasse der Copolymere, kann 0,1 bis 99,8 Gew.-%, besonders bevorzugt 0,5 bis 30 Gew.-% und insbesondere bevorzugt 1 bis 20 Gew.-% betragen.

Als polymerisationsfähige, siliziumhaltige Komponenten D) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen silikonhaltigen Monomere über die entstehenden Polymerketten hinweg nicht notwendigerweise statistisch erfolgen.
Auch die Ausbildung von beispielsweise block- (auch multiblock-) oder gradientenartigen Strukturen ist im Sinne der Erfindung. Kombinationen von zwei oder mehreren unterschiedlichen silikonhaltigen Vertretern sind auch möglich. Die Verwendung von silikonhaltigen Komponenten mit zwei oder mehr polymerisationsaktiven Gruppen führt zum Aufbau verzweigter oder vernetzter Strukturen.

Bevorzugte silikonhaltige Komponenten sind solche gemäß Formel (I).

R¹-Z-[(Si(R³R⁴)-O-)_{w}-(Si(R⁵R⁶)-O)ₓ-]-R² (I)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ einen Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest dar.
Zur Anbindung der silikonhaltigen Polymerkette an die reaktive Endgruppe R¹ ist eine geeignete chemische Brücke Z erforderlich. Bevorzugte Brücken Z sind -O-, -((C₁ - C₅₀)Alkylen)-, -((C₆ - C₃₀) Arylen)-, -((C₅ - C₈) Cycloalkylen)-, -((C₁-C₅₀)Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann. Weiterhin geeignet als Brückegruppierungen Z sind -((C₁ - C₁₀)Alkyl)-(Si(OCH₃)₂)- und -(Si(OCH₃)₂)-.
Der polymere Mittelteil wird durch silikonhaltige Wiederholungseinheiten repräsentiert.
Die Reste R³, R⁴, R⁵ und R⁶ bedeuten unabhängig voneinander -CH₃, -O-CH₃, -C₆H₅ oder -O-C₆H₅.
Die Indizes w und x repräsentieren stöchiometrische Koeffizienten, die unabhängig voneinander 0 bis 500, bevorzugt 10 bis 250, betragen.

Die Verteilung der Wiederholungseinheiten über die Kette hinweg kann nicht nur rein statistisch, sondern auch blockartig, alternierend oder gradientenartig sein kann.
R² kann einerseits einen aliphatischen, olefinischen, cycloaliphatischen, arylaliphatischen oder aromatischen (C₁-C₅₀)-Kohlenwasserstoffrest symbolisieren (linear oder verzweigt) oder -OH, -NH₂, -N(CH₃)₂, -R⁷ oder für die Struktureinheit [-Z-R¹] stehen. Die Bedeutung der beiden Variablen Z und R¹ wurde bereits erklärt. R⁷ steht für weitere Si-haltige Gruppierungen. Bevorzugte R⁷-Reste sind -O-Si(CH₃)₃, -O-Si(Ph)₃, -O-Si(O-Si(CH₃)₃)₂CH₃) und -O-Si(O-Si(Ph)₃)₂Ph).
Wenn R² ein Element der Gruppe [-Z-R¹] darstellt, handelt es sich um difunktionelle, Monomere, die zur Vernetzung der entstehenden Polymerstrukturen herangezogen werden können.
Formel (I) beschreibt nicht nur vinylisch funktionalisierte, silikonhaltige Polymerspezies mit einer polymertypischen Verteilung, sondern auch definierte Verbindungen mit diskreten Molekulargewichten.

Besonders bevorzugte silikonhaltige Komponenten sind die folgenden acrylisch- oder methacrylisch modifizierten silikonhaltigen Komponenten:

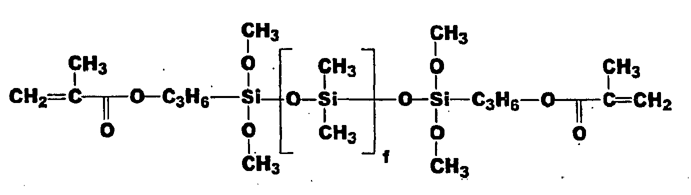

Methacryloxyproplydimethylsilyl endgeblockte Polydimethylsiloxane (f=2 bis 500)

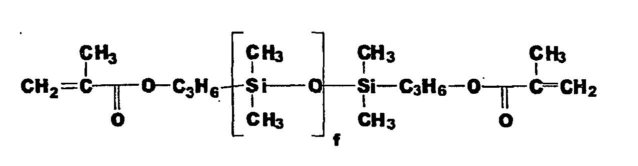

Methacryloxypropyl endgeblockte Polydimethylsiloxane (f= 2 bis 500 bis)

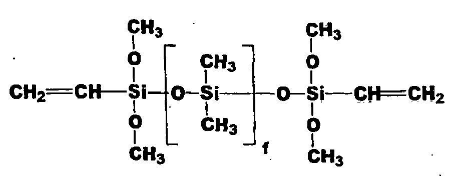

Vinyldimethoxysilyl endgeblockte Polydimethylsiloxane (f=2-500)

Bezogen auf die Gesamtmasse der Copolymere kann der Gehalt an siliziumhaltigen Komponenten bis 99.9 Gew.-%, bevorzugt 0.5 bis 30 Gew.-%, insbesondere bevorzugt 1 bis 20 Gew.-%, betragen.

Als polymerisationsfähige, fluorhaltige Komponenten E) sind alle mindestens einfach olefinisch ungesättigten Verbindungen geeignet, die unter den jeweils gewählten Reaktionsbedingungen zur radikalischen Copolymerisation befähigt sind. Dabei muss die Verteilung der einzelnen fluorhaltigen Monomere über die entstehenden Polymerketten hinweg nicht notwendigerweise statistisch erfolgen. Auch die Ausbildung von beispielsweise block- (auch multiblock-) oder gradientenartigen Strukturen ist im Sinne der Erfindung. Kombinationen von zwei oder mehreren unterschiedlichen, fluorhaltigen Komponenten E) ist auch möglich, wobei dem Experten klar ist, dass monofunktionelle Vertreter zur Bildung kammförmiger Strukturen führen, wohingegen di-, tri- oder polyfunktionelle Komponenten E) zu zumindest teilvernetzten Strukturen führen.

Bevorzugte fluorhaltige Komponenten E) sind solche gemäß Formel (II).

R¹-Y-CᵣH₂ᵣCₛF₂ₛCF₃ (II)

Dabei stellt R¹ eine polymerisationsfähige Funktion aus der Gruppe der vinylisch ungesättigten Verbindungen dar, die zum Aufbau polymerer Strukturen auf radikalischem Wege geeignet ist. Bevorzugt stellt R¹ ein Vinyl-, Allyl-, Methallyl-, Methylvinyl-, Acryl- (CH₂=CH-CO-), Methacryl- (CH₂=C[CH₃]-CO-), Crotonyl-, Senecionyl-, Itaconyl-, Maleinyl-, Fumaryl- oder Styrylrest, besonders bevorzugt einen Acryl- und Methacrylrest, dar.

Zur Anbindung der fluorhaltigen Gruppierung an die reaktive Endgruppe R¹ ist eine geeignete chemische Brücke Y erforderlich. Bevorzugte Brücken Y sind -O-, -C(O)-, -C(O)-O-, -S-, -O-CH₂-CH(O-)-CH₂OH, -O-CH₂-CH(OH)-CH₂-O-, -O-SO₂-O-,-O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und -O-([CH-CH₂-O]ₙ-[CH₂-CH₂-O]ₘ)ₒ-, wobei n, m und o unabhängig voneinander Zahlen von 0 bis 200 bedeuten und die Verteilung der EO- und PO-Einheiten statistisch oder blockförmig sein kann. Bei r und s handelt es sich um stöchiometrische Koeffizienten, die unabhängig voneinander Zahlen von 0 bis 200 bedeuten.

Bevorzugte fluorhaltige Komponenten E) gemäß Formel (II) sind
Perfluorhexylethanol-methacrylat,
Perfluorhexoylpropanol-methacrylat,
Perfluoroctyethanol-methacrylat,
Perfluoroctylpropanol-methacrylat,
Perfluorhexylethanolylpolygycolether-methacrylat,
Perfluorhexoyl-propanolyl-poly-[ethylglykol-co-propylenglycolether]-acrylat,
Perfluoroctyethanolyl-poly-[ethylglykol-blockco-propylenglycolether]-methacrylat,
Perfluoroctylpropanolyl-polypropylen-glycolether-methacrylat.

Bezogen auf die Gesamtmasse des Copolymeren kann der Gehalt an fluorhaltigen Komponenten bis 99,9 Gew.-%, bevorzugt 0,5 bis 30 Gew.-%, insbesondere bevorzugt 1 bis 20 Gew.-%, betragen.

Bei den Makromonomeren F) acrylisch- oder methacrylisch monofunktionalisierte Alkylethoxylate gemäß Formel (IV). 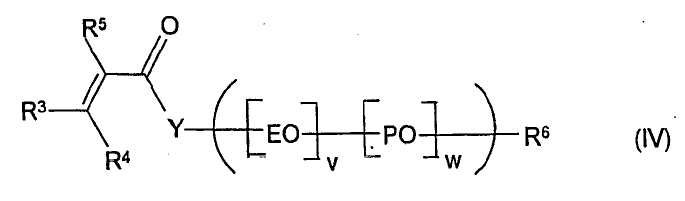 R₃, R₄, R₅ und R₆ bedeuten unabhängig voneinander Wasserstoff oder n-aliphatische, iso-aliphatische, olefinische, cycloaliphatische, arylaliphatische oder aromatische (C₁-C₃₀)-Kohlenwasserstoffreste.
Bevorzugt sind R₃ und R₄ gleich H oder -CH₃, besonders bevorzugt H; R₅ ist gleich H oder -CH₃; und R₆ ist gleich einem n-aliphatischen, iso-aliphatischen, olefinischen, cycloaliphatischen, arylaliphatischen oder aromatischen (C₁-C₃₀)-Kohlenwasserstoffrest.
v und w sind wiederum die stöchiometrischen Koeffizienten betreffend die Ethylenoxideinheiten (EO) und Propylenoxideinheiten (PO). v und w betragen unabhängig voneinander 0 bis 500, bevorzugt 1 bis 30, wobei die Summe aus v und w im Mittel ≥ 1 sein muss. Die Verteilung der EO- und PO-Einheiten über die Makromonomerkette kann statistisch, blockartig, alternierend oder gradientenartig sein. Y steht für die obengenannten Brücken.

Weiterhin insbesondere bevorzugte Makromonomeren F) haben die folgende Struktur gemäß Formel (IV):

| Bezeichnung | R³ | R⁴ | R⁵ | R⁶ | v | w |
|---|---|---|---|---|---|---|
| ^{®}LA-030-methyacrylat | H | H | -CH₃ | -Lauryl | 3 | 0 |
| ^{®}LA-070-methacrylat | H | H | -CH₃ | -Lauryl | 7 | 0 |
| ^{®}LA-200-methacrylat | H | H | -CH₃ | -Lauryl | 20 | 0 |
| ^{®}LA-250-methacrylat | H | H | -CH₃ | -Lauryl | 25 | 0 |
| ^{®}T-080-methyacrylat | H | H | -CH₃ | -Talk | 8 | 0 |
| ^{®}T-080-acrylat | H | H | H | -Talk | 8 | 0 |
| ^{®}T-250-methyacrylat | H | H | -CH₃ | -Talk | 25 | 0 |
| ^{®}T-250-crotonat | -CH₃ | H | -CH₃ | -Talk | 25 | 0 |
| ^{®}OC-030-methacrylat | H | H | -CH₃ | -Octyl | 3 | 0 |
| ^{®}OC-105-methacrylat | H | H | -CH₃ | -Octyl | 10 | 5 |
| ^{®}Behenyl-010-methyaryl | H | H | H | -Behenyl | 10 | 0 |
| ^{®}Behenyl-020-methyaryl | H | H | H | -Behenyl | 20 | 0 |
| ^{®}Behenyl-010-senecionyl | -CH₃ | -CH₃ | H | -Behenyl | 10 | 0 |
| ^{®}PEG-440-diacrylat | H | H | H | -Acryl | 10 | 0 |
| ^{®}B-11-50-methacrylat | H | H | -CH₃ | -Butyl | 17 | 13 |
| ^{®}MPEG-750-methacrylat | H | H | -CH₃ | -Methyl | 18 | 0 |
| ^{®}P-010-acrylat | H | H | H | -Phenyl | 10 | 0 |
| ^{®}O-050-acrylat | H | H | H | -Oleyl | 5 | 0 |

Weiterhin als Makromonomere F) insbesondere geeignet sind Ester der (Meth)acrylsäure mit
(C₁₀-C₁₈)-Fettalkoholpolyglykolethern mit 8 EO-Einheiten (Genapol^{®} C-080)
C₁₁-Oxoalkoholpolyglykolethern mit 8 EO-Einheiten (Genapol^{®} UD-080)
(C₁₂-C₁₄)-Fettalkoholpolyglykolethern mit 7 EO-Einheiten (Genapol^{®} LA-070)
(C₁₂-C₁₄)-Fettalkoholpolyglykolethern mit 11 EO-Einheiten (Genapol^{®} LA-110)
(C₁₆-C₁₈)-Fettalkoholpolyglykolethern mit 8 EO-Einheiten (Genapol^{®} T-080)
(C₁₆-C₁₈)-Fettalkoholpolyglykolethern mit 15 EO-Einheiten (Genapol^{®} T-150)
(C₁₆-C₁₈)-Fettalkoholpolyglykolethern mit 11 EO-Einheiten (Genapol^{®} T-110)
(C₁₆-C₁₈)-Fettalkoholpolyglycolethern mit 20 EO-Einheiten (Genapol^{®} T-200)
(C₁₆-C₁₈)-Fettalkoholpolyglycolethern mit 25 EO-Einheiten (Genapol^{®} T-250) (C₁₈-C₂₂)-Fettalkoholpolyglycolethern mit 25 EO-Einheiten und/oder
iso-(C₁₆-C₁₈)-Fettalkoholpolyglycolethern mit 25 EO-Einheiten
Bei den Genapol^{®}-Typen handelt es sich um Produkte der Firma Clariant, GmbH.

Bevorzugt beträgt das Molekulargewicht der Makromonomeren F) 200 g/mol bis 10⁶ g/mol, besonders bevorzugt 150 bis 10⁴ g/mol und insbesondere bevorzugt 200 bis 5000 g/mol.

Bezogen auf die Gesamtmasse der Copolymere können geeignete Makromonomere bis zu 99,9 Gew.-% eingesetzt werden. Bevorzugt finden die Bereiche 0,5 bis 30 Gew.-% und 70 bis 99,5 Gew.-% Anwendung. Besonders bevorzugt sind Anteile von 1 bis 20 Gew.-% und 75 bis 95 Gew.-%.

Weiterhin bevorzugt als Copolymere sind solche, die durch Copolymerisation mindestens der Komponenten A) C) und F) erhältlich sind.

Weiterhin bevorzugt als Copolymere sind solche, die durch Copolymerisation mindestens der Komponenten A), D) und F) erhältlich sind.

Weiterhin bevorzugt als Copolymere sind solche, die durch Copolymerisation mindestens der Komponenten A) und F) erhältlich sind.

In einer bevorzugten Ausführungsform wird die Copolymerisation in Gegenwart mindestens eines polymeren Additivs G) durchgeführt, wobei das Additiv G) vor der eigentlichen Copolymerisation dem Polymerisationsmedium ganz- oder teilweise gelöst zugegeben wird. Die Verwendung von mehreren Additiven G) ist ebenfalls erfindungsgemäß. Vernetzte Additive G) können ebenfalls verwendet werden.
Die Additive G) bzw. deren Mischungen müssen lediglich ganz oder teilweise im gewählten Polymerisationsmedium löslich sein. Während des eigentlichen Polymerisationsschrittes hat das Additiv G) mehrere Funktionen. Einerseits verhindert es im eigentlichen Polymerisationsschritt die Bildung übervernetzter Polymeranteile im sich bildenden Copolymerisat und andererseits wird das Additiv G) gemäß dem allgemein bekannten Mechanismus der Pfropfcopolymerisation statistisch von aktiven Radikalen angegriffen. Dies führt dazu, dass je nach Additiv G) mehr oder weniger große Anteile davon in die Copolymere eingebaut werden. Zudem besitzen geeignete Additive G) die Eigenschaft, die Lösungsparameter der sich bildenden Copolymere während der radikalischen Polymerisationsreaktion derart zu verändern, dass die mittleren Molekulargewichte zu höheren Werten verschoben werden. Verglichen mit analogen Copolymeren, die ohne den Zusatz der Additive G) hergestellt wurden, zeigen solche, die unter Zusatz von Additiven G) hergestellt wurden, vorteilhafterweise eine signifikant höhere Viskosität in wässriger Lösung.
Bevorzugt als Additive G) sind in Wasser und/oder Alkoholen, bevorzugt in t-Butanol, lösliche Homo- und Copolymere. Unter Copolymeren sind dabei auch solche mit mehr als zwei verschiedenen Monomertypen zu verstehen. Besonders bevorzugt als Additive G) sind Homo- und Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxyethylmethacrylat, Diallyldimethylammoniumchlorid (DADMAC) und/oder [2-(Methacryloyloxy)ethyl]trimethylammoniumchlorid (MAPTAC); Polyalkylenglykole und/oder Alkylpolyglykole.
Insbesondere bevorzugt als Additive G) sind Polyvinylpyrrolidone (z.B. Luviskol K15^{®}, K20^{®} und K30^{®} von BASF), Poly(N-Vinylformamide), Poly(N-Vinylcaprolactame) und Copolymere aus N-Vinylpyrrolidon, N-Vinylformamid und/oder Acrylsäure, die auch teilweise oder vollständig verseift sein können. Das Molekulargewicht der Additive G) beträgt bevorzugt 10² bis 10⁷ g/mol, besonders bevorzugt 0,5*10⁴ bis 10⁶ g/mol.

Die Einsatzmenge des polymeren Additivs G) beträgt, bezogen auf die Gesamtmasse der bei der Copolymerisation zu polymerisierenden Monomere, bevorzugt 0,1 bis 90 Gew.-%, besonders bevorzugt 1 bis 20 Gew.-% und insbesondere bevorzugt 1,5 bis 10 Gew.-%.

In einer weiteren bevorzugten Ausführungsform sind die erfindungsgemäßen Copolymere vernetzt, d.h. sie enthalten Comonomere mit mindestens zwei polymerisationsfähigen Vinylgruppen.
Bevorzugte Vernetzer sind Methylenbisacrylamid; Methylenbismethacrylamid; Ester ungesättigter Mono- und Polycarbonsäuren mit Polyolen, bevorzugt Diacrylate und Triacrylate bzw. -methacrylate, besonders bevorzugt Butandiol- und Ethylenglykoldiacrylat bzw. -methacrylat, Trimethylolpropantriacrylat (TMPTA) und Trimethylolpropantrimethacrylat (TMPTMA); Allylverbindungen, bevorzugt Allyl(meth)acrylat, Triallylcyanurat, Maleinsäurediallylester, Polyallylester, Tetraallyloxyethan, Triallylamin, Tetraallylethylendiamin; Allylester der Phosphorsäure; und/oder Vinylphosphonsäurederivate.
Insbesondere bevorzugt als Vernetzer ist Trimethylolpropantriacrylat (TMPTA).

Der Gewichtsanteil an vernetzenden Comonomeren beträgt, bezogen auf die Gesamtmasse der Copolymere, bevorzugt bis 20 Gew.-%, besonders bevorzugt 0,05 bis 10 Gew.-% und insbesondere bevorzugt 0,1 bis 7 Gew.-%.

Als Polymerisationsmedium können alle organischen oder anorganischen Lösungsmittel dienen, die sich bezüglich radikalischer Polymerisationsreaktionen weitestgehend inert verhalten und vorteilhafterweise die Bildung mittlerer oder hoher Molekulargewichte zulassen. Bevorzugt Verwendung finden Wasser; niedere Alkohole; bevorzugt Methanol, Ethanol, Propanole, iso-, sec.- und t-Butanol, insbesondere bevorzugt t-Butanol; Kohlenwasserstoffe mit 1 bis 30 Kohlenstoffatomen und Mischungen der vorgenannten Verbindungen.

Die Polymerisationsreaktion erfolgt bevorzugt im Temperaturbereich zwischen 0 und 150°C, besonders bevorzugt zwischen 10 und 100°C, sowohl bei Normaldruck als auch unter erhöhtem oder erniedrigtem Druck. Gegebenenfalls kann die Polymerisation auch unter einer Schutzgasatmosphäre, vorzugsweise unter Stickstoff, ausgeführt werden.

Zur Auslösung der Polymerisation können energiereiche elektromagnetische Strahlen, mechanische Energie oder die üblichen chemischen Polymerisationsinitiatoren, wie organische Peroxide, z.B. Benzoylperoxid, tert.-Butylhydroperoxid, Methylethylketonperoxid, Cumolhydroperoxid, Dilauroylperoxid oder Azoinitiatoren, wie z.B. Azodüsobutyronitril (AIBN), verwendet werden.
Ebenfalls geeignet sind anorganische Peroxyverbindungen, wie z.B, (NH₄)₂S₂O₈, K₂S₂O₈ oder H₂O₂, gegebenenfalls in Kombination mit Reduktionsmitteln (z.B. Natriumhydrogensulfit, Ascorbinsäure, Eisen(II)-sulfat etc.) oder Redoxsystemen, welche als reduzierende Komponente eine aliphatische oder aromatische Sulfonsäure (z.B. Benzolsulfonsäure, Toluolsulfonsäure etc.) enthalten.

Als Polymerisationsmedium können alle Lösungsmittel dienen, die sich bezüglich radikalischer Polymerisationsreaktionen weitestgehend inert verhalten und die Bildung hoher Molekulargewichte zulassen. Bevorzugt Verwendung finden Wasser und niedere, tertiäre Alkohole oder Kohlenwasserstoffe mit 3 bis 30 C-Atomen. In einer besonders bevorzugten Ausführungsweise wird t-Butanol als Reaktionsmedium verwendet. Mischungen aus zwei- oder mehreren Vertretern der beschriebenen potentiellen Lösungsmitteln sind selbstverständlich ebenfalls erfindungsgemäß. Dies schließt auch Emulsionen von nicht miteinander mischbaren Solventien ein (z.B. Wasser / Kohlenwasserstoffe). Grundsätzlich sind alle Arten der Reaktionsführung geeignet, die zu den erfindungsgemäßen Polymerstrukturen führen (Lösungspolymerisation, Emulsionsverfahren, Fällungsverfahren, Hochdruckverfahren, Suspensionsverfahren, Substanzpolymerisation, Gelpolymerisation usw.).
Bevorzugt eignet sich die Fällungspolymerisation, besonders bevorzugt die Fällungspolymerisation in tert.-Butanol.

Die nachfolgende Auflistung zeigt 67 Copolymere, die für die erfindungsgemäßen Verwendungen besonders geeignet sind. Die verschiedenen Copolymere Nr. 1 bis Nr. 67 sind gemäß den folgenden Herstellverfahren 1, 2, 3 und 4 erhältlich.

### Verfahren 1:

Diese Polymere sind nach dem Fällungsverfahren in tert. Butanol herstellbar. Dabei wurden die Monomere in t-Butanol vorgelegt, die Reaktionsmischung inertisiert und anschließend die Reaktion nach Anheizen auf 60°C durch Zugabe des entsprechenden t-Butanol löslichen Initiators (bevorzugt Dilauroylperoxid) gestartet. Die Polymere werden nach beendeter Reaktion (2 Stunden) durch Absaugen des Lösungsmittels und durch anschließende Vakuumtrocknung isoliert.

### Verfahren 2:

Diese Polymere sind nach dem Gelpolymerisationsverfahren in Wasser herstellbar. Dabei werden die Monomere in Wasser gelöst, die Reaktionsmischung inertisiert und anschließend die Reaktion nach Anheizen auf 65°C durch Zugabe von geeigneten Initiatoren- oder Initiatorsystemen (bevorzugt Na₂S₂O₈) gestartet. Die Polymergele werden anschließend zerkleinert und nach Trocknung die Polymere isoliert.

### Verfahren 3:

Diese Polymere sind nach dem Emulsionsverfahren in Wasser herstellbar. Dabei werden die Monomere in einer Mischung aus Wasser/organ. Lösungsmittel (bevorzugt Cyclohexan) unter Verwendung eines.Emulgators emulgiert, die Reaktionsmischung mittels N₂ inertisiert und anschließend die Reaktion nach Anheizen auf 80°C durch Zugabe von geeigneten Initiatoren- oder Initiatorsystemen (bevorzugt Na₂S₂O₈) gestartet. Die Polymeremulsionen werden anschließend eingedampft (Cyclohexan fungiert als Schlepper für Wasser) und dadurch die Polymere isoliert.

### Verfahren 4:

Diese Polymere sind nach dem Lösungsverfahren in organischen Lösungsmitteln (bevorzugt Toluol, z.B. auch tert. Alkohole) herstellbar. Dabei werden die Monomere im Lösungsmittel vorgelegt, die Reaktionsmischung inertisiert und anschließend die Reaktion nach Anheizen auf 70°C durch Zugabe von geeigneten Initiatoren- oder Initiatorsystemen (bevorzugt Dilauroylperoxid) gestartet. Die Polymere werden durch Abdampfen des Lösungsmittels und durch anschließende Vakuumtrocknung isoliert.

Polymere mit hydrophoben Seitenketten, unvernetzt

| Nr. | Zusammensetzung | Herstellverfahren |
|---|---|---|
| 1 | 95 g AMPS 5 g Genapol T-080 | 1 |
| 2 | 90 g AMPS 10 g Genapol T-080 | 1 |
| 3 | 85 g AMPS 15 g Genapol T-080 | 1 |
| 4 | 80 g AMPS 20 g Genapol T-080 | 1 |
| 5 | 70 g AMPS 30 g Genapol T-080 | 1 |
| 6 | 50 g AMPS 50 g Genapol T-080 | 3 |
| 7 | 40 g AMPS 60 g Genapol T-080 | 3 |
| 8 | 30 g AMPS 70 g Genapol T-080 | 3 |
| 9 | 20 g AMPS 80 g Genapol T-080 | 3 |
| 10 | 60 g AMPS 60 g BB10 | 4 |
| 11 | 80 g AMPS 20 g BB10 | 4 |
| 12 | 90 g AMPS 10 g BB10 | 3 |
| 13 | 80 g AMPS 20gBB10 | 1 |
| 14 | 80 g AMPS 20 g Genapol LA040 | 1 |

Polymere mit hydrophoben Seitenketten, vernetzt

| Nr. | Zusammensetzung | Herstellverfahren |
|---|---|---|
| 15 | 80 g AMPS 20 g Genapol LA040 0.6g AMA | 1 |
| 16 | 80 g AMPS 20 g Genapol LA040 0,8 AMA | 1 |
| 17 | 80 g AMPS 20 g Genapol LA040 1,0 g AMA | 1 |
| 18 | 628,73 g AMPS 120,45 g Genapol T-250 6,5 g TMPTA | 2 |
| 19 | 60 g AMPS 40 g BB10 1,9 g TMPTA | 4 |
| 20 | 80 g AMPS 20 g BB10 1,4 g TMPTA | 4 |
| 21 | 90 g AMPS 10 g BB10 1,9 g TMPTA | 4 |
| 22 | 80 g AMPS 20 g BB10 1,9 g TMPTA | 4 |
| 23 | 60 g AMPS 40 g BB10 1,4 g TMPTA | 4 |

Polymere mit hydrophoben Seitenketten, vernetzt, gepfropft

| Nr. | Zusammensetzung | Herstellverfahren |
|---|---|---|
| 24 | 95 g AMPS 5 g BB10, 1,9 g TMPTA, 1 g Poly-NVP | 1 |
| 25 | 90 g AMPS 10 g BB10, 1,9 g TMPTA, 1 g Poly-NVP | 1 |
| 26 | 85 g AMPS 15 g BB10, 1,9 g TMPTA, 1 g Poly-NVP | 1 |
| 27 | 90 g AMPS 10 g BB10, 1,9 g TMPTA, 1 g Poly-NVP | 1 |

Polymere mit siliziumhaltigen Gruppen, unvernetzt

| Nr. | Zusammensetzung | Herstellverfahren |
|---|---|---|
| 28 | 80 g AMPS, 20 g Silvet 867 | 1 |
| 29 | 80 g AMPS, 50 g Silvet 867 | 4 |

Polymere mit siliziumhaltigen Gruppen, vernetzt

| Nr. | Zusammensetzung | Herstellverfahren |
|---|---|---|
| 30 | 80 g AMPS, 20 g Silvet 867, 0,5 g MBA | 4 |
| 31 | 80 g AMPS, 20 g Silvet 867, 1,0 g MBA | 1 |
| 32 | 60 g AMPS, 40 g Y-12867, 0,95 g AMA | 1 |
| 33 | 80 g AMPS, 20 g Y-12867, 0,95 g AMA | 1 |
| 34 | 90 g AMPS, 10 g Y-12867, 0,95 g AMA | 1 |
| 35 | 60 g AMPS, 40 g Silvet 7280, 0,95 g AMA | 1 |
| 36 | 80 g AMPS, 20 g Silvet 7280, 0,95 g AMA | 1 |
| 37 | 90 g AMPS, 10 g Silvet 7280, 0,95 g AMA | 1 |
| 38 | 60 g AMPS, 40 g Silvet 7608, 0,95 g AMA | 1 |
| 39 | 80 g AMPS, 20 g Silvet 7608, 0,95 g AMA | 1 |
| 40 | 90 g AMPS, 10 g Silvet 7608, 0,95 g AMA | 1 |

Polymere mit hydrophoben Seitenketten und kationischen Gruppen, unvernetzt

| Nr. | Zusammensetzung | Herstellverfahren |
|---|---|---|
| 41 | 87,5 g AMPS, 7,5 g Genapol T-110, 5 g DADMAC | 2 |
| 42 | 40 g AMPS, 10 g Genapol T110, 45 g Methacrylamid | 2 |
| 43 | 55 g AMPS, 40 g Genapol LA040, 5 g Quat | 1 |
| 44 | 75 g AMPS, 10 g BB10, 6,7 g Quat | 1 |

Polymere mit hydrophoben Seitenketten und kationischen Gruppen, vernetzt

| Nr. | Zusammensetzung | Herstellverfahren |
|---|---|---|
| 45 | 60 g AMPS, 20 g Genapol T-80, 10 g Quat, 10 g HEMA | 1 |
| 46 | 75 g AMPS, 20 g GenapolT-250, 5 g Quat, 1,4 g TMPTA | 1 |
| 47 | 75 g AMPS, 20 g GenapolT-250, 10 g Quat, 1,4 g TMPTA | 1 |
| 48 | 75 g AMPS, 20 g GenapolT-250, 20 g Quat, 1,4 g TMPTA | 1 |

Polymere mit fluorhaltigen Gruppen

| Nr. | Zusammensetzung' | Herstellverfahren |
|---|---|---|
| 49 | 94 g AMPS, 2,02 g Fluowet AC 600 | 1 |
| 50 | 80 g AMPS, | 3 |
| | 20 g Perfluoroctylpolyethylenglykolmethacrylat, 1 g Span 80 | |

Polymere mit fluorhaltigen Gruppen, gepfropft

| Nr. | Zusammensetzung | Herstellverfahren |
|---|---|---|
| 51 | 80 g AMPS, 10 g Fluowet AC 600, 5 g Poly-NVP | 1 |
| 52 | 70 g AMPS, 8 g Perfluoroctylethyloxyglycerinmethacrylat, | 4 |
| | 5 g Poly-NVP | |

Multifunktionelle Polymere

| Nr. | Zusammensetzung | Herstellverfahren |
|---|---|---|
| 53 | 80 g AMPS, 10 g Genapol LA070, 10 g Silvet 7608, | 1 |
| | 1,8 g TMPTA | |
| 54 | 70 g AMPS, 5 g N-Vinylpyrrolidon, 15 g Genapol T-250 methacrylat, 10 g Quat, 10 g Poly-NVP | 4 |
| 55 | 80 g AMPS, 5 g N-Vinylformamid, 5 g Genapol O-150-methacrylat, 10 g DADMAC, 1,8 g TMPTA, 8 g Poly-N-Vinylformamid | 2 |
| 56 | 70 g AMPS, 5 g N-Vinylpyrrolidon, 15 g Genapol T-250-methacrylat, 10 g Quat, 10 g Poly-NVP | 1 |
| 57 | 60 g AMPS, 10 g Genapol-BE-020-methacrylat, | 1 |
| | 10 g Genapol T-250-acrylat, 20 g Quat, 1 g Span 80 | |
| 58 | 60 g AMPS, 20 g MPEG-750-methacrylat, | 1 |
| | 10 g Methacryloxypyldimethicon, | |
| | 10 g Perfluorooctylpolyethylenglycol-methacrylat, | |
| | 10 g Poly[N-vinylcaprolacton-co-acrylsäure] (10/90) | |
| 59 | 80 g AMPS, 5 g N-Vinylformamid, 5 g Genapol O-150-methacrylat, 10 g DADMAC, 1,8 g TMPTA | 1 |
| 60 | 70 g AMPS, 10 g Genapol T-250-acrylat, | 1 |
| | 5 g N-Methyl-4-vinylpyridiniumchlorid, | |
| | 2,5 g Silvet Y-12867, | |
| | 2,5 g Perfluorhexylpolyethylenglykolmethacrylat, | |
| | 10 g Polyethylenglykoldimethacrylat, | |
| | 4 g Poly[N-Vinylcaprolactam] | |
| 61 | 10 g AMPS, 20 g Acrylamid, 30 g N-2-Vinylpyrrolidon, | 3 |
| | 20 g Silvet 7608, 10 g Methacryloxypyl dimethicon, | |
| | 10 g Fluowet AC 812 | |
| 62 | 60 g AMPS, 10 g DADMAC, 10 g Quat, 10 g Genapol-LA-250-crotonat, 10 g Methacryloxypyldimethicon, | 1 |
| | 7 g Poly[acrylsäure-co-N-vinylformamid] | |
| 63 | 50 g AMPS, 45 g Silvet 7608, 1,8 g TMPTA, | 1 |
| | 8 g Poly[N-Vinylformamid] | |
| 64 | 20 g AMPS, 10 g Genapol T 110, 35 g MAA, 30 g | 4 |
| | HEMA, 5 g DADMAC | |
| 65 | 20 g AMPS, 80 g BB10, 1,4 g TMPTA | 1 |
| 66 | 75 g AMPS, 20 g BB10, 6,7 g Quat, 1,4 g TMPTA | 1 |
| 67 | 35 g AMPS, 60 g Acrylamid, 2 g VIFA, | 4 |
| | 2,5 g Vinylphosphonsäure, 2 Mol-% Fluowet EA-600 | |

Chemische Bezeichnung der Reaktanden:

| | |
|---|---|
| AMPS | Acryloyldimethyltaurat, wahlweise Na- oder NH4-Salz |
| Genapol^{®} T-080 | C₁₆-C₁₈-Fettalkoholpolyglykolether mit 8 EO-Einheiten |
| Genapol^{®} T-110 | C₁₂-C₁₄-Fettalkoholpolyglykolether mit 11 EO -Einheiten |
| Genapol^{®} T-250 | C₁₆-C₁₈-Fettalkoholpolyglycolether mit 25 EO-Einheiten |
| Genapol^{®} LA-040 | C₁₂-C₁₄-Fettalkoholpolyglykolether mit 4 EO-Einheiten |
| Genapol^{®} LA-070 | C₁₂-C₁₄-Fettalkoholpolyglykolether mit 7 EO-Einheiten |
| Genapol^{®} O-150 methacrylat | C₁₆-C₁₈-Fettalkoholpolyglykolether methacrylat mit 15 EO-Einheiten, |
| Genapol^{®} LA-250 crotonat | C₁₂-C₁₄-Fettalkoholpolyglykolether crotonat mit 25 EO-Einheiten |
| Genapol^{®} T-250 methacrylat | C₁₆-C₁₈-Fettalkoholpolyglycolether methacrylat mit 25 EO-Einheiten |
| Genapol^{®} T-250 acrylat | C₁₆-C₁₈-Fettalkoholpolyglycolether methacrylat mit 25 EO-Einheiten |
| BB10^{®} | Polyoxyethylen(10)Behenylether |
| TMPTA | Trimethylolpropantriacrylat |
| Poly-NVP | Poly-N-Vinylpyrrolidon |
| Silvet^{®} 867 | Siloxan Polyalkylenoxid Copolymer |
| MBA | Methylen-bis-acrylamid |
| AMA | Allylmethacrylat |
| ^{®}Y-12867 | Siloxan Polyalkylenoxid Copolymer |
| Silvet^{®} 7608 | Polyalkylenoxid-modifiziertes |
| Heptamethyltrisiloxan | |
| Silvet^{®} 7280 | Polyalkylenoxid-modifiziertes |
| Heptamethyltrisiloxan | |
| DADMAC | Diallyldimethyl-ammoniumchlorid |
| HEMA | 2-Hydroxyethylmethacrylat |
| Quat | 2-(Methacryloyloxy)ethyltrimethylammoniumchlorid |
| Fluowet^{®} AC 600 | Perfluoralkylethylacrylat |
| Span^{®} 80 | Sorbitanester |

In einer bevorzugten Ausführungsform sind die Copolymere wasserlöslich oder wasserquellbar.

Die beschriebene, optional durchführbare Pfropfung der Copolymere mit anderen Polymeren führt zu Produkten mit besonderer Polymermorphologie, die in wässrigen Systemen optisch klare Gele ergeben. Ein potenzieller Nachteil der Copolymere ohne Pfropfung besteht in einer mehr oder weniger starken Opaleszenz in wässriger Lösung. Diese beruht auf bisher nicht zu vermeidenden, übervernetzten Polymeranteilen, die während der Synthese entstehen und in Wasser nur unzureichend gequollen vorliegen. Dadurch bilden sich Licht streuende Teilchen aus, deren Größe deutlich oberhalb der Wellenlänge des sichtbaren Lichts liegt und deshalb Ursache der Opaleszenz sind. Durch das beschriebene, optional durchführbare Pfropfverfahren wird die Bildung übervernetzter Polymeranteile gegenüber herkömmlichen Techniken deutlich reduziert oder gänzlich vermieden.
Die beschriebene, optional durchführbare Inkorporation sowohl von kationischen Ladungen als auch von Silizium-, Fluor oder Phosphoratomen in die Copolymere führt zu Produkten, die in kosmetischen Formulierungen besondere sensorische und rheologische Eigenschaften besitzen. Eine Verbesserung der sensorischen und rheologischen Eigenschaften kann insbesondere bei der Verwendung in rinse off Produkten (insbesondere Haarbehandlungsmittel) als auch leave on Produkten (insbesondere O/W Emulsionen) gewünscht sein.

Siliziummodifizierte Copolymere können die Funktionen von Silikonölen in teilweise oder in vollem Umfang übernehmen. Der Einsatz von Silikonen kann durch die Copolymere reduziert oder vermieden werden.

Vorteilhafte Eigenschaften zeigen die Copolymere sowohl in vernetzter als auch in unvernetzter Form. Während vernetzte Systeme z.B. hervorragende Eigenschaftsprofile im Hinblick auf Emulsionsstabilisierung zeigten, konnten insbesondere mit Hilfe der unvernetzten Varianten tensidhaltige Lösungen verdickt werden. Gleiches gilt für elektrolythaltige Systeme, die bekanntermaßen mit Polyelektrolyten nur sehr schwer oder gar nicht zu verdicken sind.

Die Copolymere können als Verdicker für Mittel auf wässriger oder wässrigalkoholischer Basis, beispielsweise Haargele, eingesetzt werden. Des weiteren eignen sich die Copolymere als Stabilisator, Dispergiermittel und Konsistenzgeber für wässrig-tensidische Zubereitungen, beispielsweise Shampoos, Duschbäder, Duschgels, Schaumbäder und dergleichen.
Die verdickende Wirkung der Copolymere in wässrig-tensidischen Mitteln wird durch eine Assoziation der Polymerseitenketten und der Tenside verstärkt und kann durch die Wahl der Seitenketten der Copolymere und durch die Wahl der Tenside gesteuert werden.

Die suspendierende bzw. dispergierende und stabilisierende Wirkung der Copolymere in wässrig-tensidischen Mitteln wird durch die Assoziation der Polymerseitenketten bzw. funktionellen Gruppen in Haupt- und Seitenkette und der in wässrig-tensidischen Mitteln unlöslichen flüssigen Komponenten, beispielsweise Silikonöle, bzw. der unlöslichen Komponenten, beispielsweise Zink-Pyrethione, bedingt.
Die Copolymere eignen sich ebenso als Verdicker und Dispergiermittel, als Emulgatoren, Suspendiermittel mit verdickender Wirkung und Konsistenzgeber für Emulsionen und Suspensionen, sowie als Gleitmittel, Haftmittel, Verdicker, Dispergier- und Emulgiermittel dekorativer, feststoffhaltiger Zubereitungen. Dabei können auch Mischungen der Copolymere verwendet werden. Die emulgierende, stabilisierende und/oder konsistenzgebende Wirkung der Copolymere in Emulsionen wird durch eine Assoziation der Polymerseitenketten untereinander, sowie durch eine Wechselwirkung der Polymerseitenketten mit den hydrophoben Ölkomponenten verursacht bzw. verstärkt.

Die Copolymere können sowohl in Wasser-in-Öl-Emulsionen als auch Öl-in-Wasser-Emulsionen, Mikroemulsionen und multiple Emulsionen verwendet werden.
Die Herstellung der Emulsionen kann in bekannter Weise, d.h. beispielsweise durch Heiß-, Heiß/Kalt- bzw. PIT-Emulgierung erfolgen.
Der nichtwässrige Anteil der Emulsionen, der sich weitgehend aus dem Emulgator, dem Verdicker und dem Ölkörper zusammensetzt, liegt üblicherweise bei 5 bis 95 Gew.-%, vorzugsweise bei 15 bis 75 Gew.-%. Daraus folgt, dass die Emulsionen 5 bis 95 Gew.-% und vorzugsweise 25 bis 85 Gew.-% Wasser enthalten können, abhängig davon, ob Lotionen mit einer vergleichsweise niedrigen oder Cremes und Salben mit hoher Viskosität hergestellt werden sollen.

Die Copolymere eignen sich für den Einsatz in Rinse-off Produkten, beispielsweise Shampoos, Duschbäder, Duschgels, Schaumbäder, aber auch in leave-on Produkten, beispielsweise Hautpflegemitteln, Tagescremes, Nachtcremes, Pflegecremes, Nährcremes, Bodylotions, Salben, Sonnenschutzmitteln, Lippenpflegemitteln und Deodorantien. Des weiteren eignen sie sich auch für tensidfreie wässrige Mittel und Emulsionen, beispielsweise für Haarkuren und -spülungen, Haargele aber auch für Dauerwellenmittel, Haarfärbemittel, sowie für dekorative Kosmetika, beispielsweise make-ups, eyeshadows, Lippenstifte, Mascara und dergleichen.

Bevorzugt als Verdicker und Konsistenzgeber eignen sich insbesondere vernetzte Copolymere. Bevorzugt als Verdicker und Konsistenzgeber sind ferner solche Copolymere, die durch Copolymerisation der Komponenten A) und F) erhältlich sind.
Weiterhin bevorzugt als Copolymere sind solche, die durch Copolymerisation der Komponenten A), C) und F) erhältlich sind.
Je nach Substitutionsgrad lässt sich die Wasser- bzw. die Ölverträglichkeit einstellen. Bevorzugt als Verdicker und Konsistenzgeber für wässrige (hydrophile) Systeme sind solche Copolymere mit einem Anteil der Komponente A) von 1 bis 50 Gew.-%.
Bevorzugt als Verdicker und Konsistenzgeber für hydrophobe Systeme sind solche Copolymere mit einem Anteil der Komponente A) von 50 bis 98 Gew.-%.

Besonders bevorzugt ist die Verwendung als Verdicker und Konsistenzgeber in leave-on Produkten, beispielsweise in Hautpflegemitteln, Tagescremes, Nachtcremes, Pflegecremes, Nährcremes, Bodylotions, Salben, Sonnenschutzmitteln, Lippenpflegemitteln und Deodorantien. Des weiteren eignen sie sich auch für tensidfreie wässrige Mittel und Emulsionen, beispielsweise für Haargele oder Gel-Cremes.

Weiterhin bevorzugt als Copolymere sind solche, die durch Copolymerisation der Komponenten A) C) und F) erhältlich sind.
Weiterhin bevorzugt als Copolymere sind solche, die durch Copolymerisation der Komponenten A), D) und F) erhältlich sind.

Weiterhin bevorzugt als Copolymere sind solche, die durch Copolymerisation der Komponenten A) und F) erhältlich sind.

Der Einbau von kationischen Komponenten C) erhöht die Substantivität der Copolymere für Haar- und Hautoberflächen und verbessert somit die pflegenden Eigenschaften. Der Einbau von Silikon-Komponenten D) führt zu einer Copolymere mit pflegenden Eigenschaften und verbesserten sensorischen Profilen.

Besonders bevorzugt ist die Verwendung solcher Copolymere als Conditioner in Hautpflegemitteln und insbesondere in Haarpflegemitteln in Form von rinse-off Produkten, beispielsweise Shampoos, Duschbäder, Duschgels, Schaumbäder.

Bevorzugt als Dispergatoren und Emulgatoren eignen sich solche Copolymere, die durch Copolymerisation der Komponenten A) und F) erhältlich sind.
Die Si-Derivatisierung macht die so erhaltenen Emulgatoren insbesondere für Silikonöle geeignet.
Weiterhin bevorzugt als Copolymere sind solche, die durch Copolymerisation der Komponenten A), C) und F) erhältlich sind.
Je nach Substitutionsgrad lässt sich die Wasser- bzw. die Ölverträglichkeit einstellen.
Bevorzugt als Öl-in-Wasser-Emulgatoren sind solche Copolymere mit einem Anteil der Komponente A) von 1 bis 50 %.
Bevorzugt als Wasser-in-Öl-Emulgatoren sind solche Copolymere mit einem Anteil der Komponente A) von 50 bis 98 %.

Besonders bevorzugt ist die Verwendung als Emulgator in leave-on Produkten, beispielsweise Hautpflegemitteln, Tagescremes, Nachtcremes, Pflegecremes, Nährcremes, Bodylotions, Salben, Sonnenschutzmitteln, Lippenpflegemitteln und Deodorantien.

Besonders bevorzugt ist die Verwendung als Dispergator rinse-off Produkten, enthaltend unlösliche flüssige (z.B. Öle) oder feste (z.B. Perlglanzmittel oder Antischuppenwirkstoffe) Komponenten, beispielsweise in Shampoos, Duschbäder, Duschgels, Schaumbäder.

Bei der erfindungsgemäßen Verwendung in wässrigen, wässrig-alkoholischen oder wässrig-tensidischen Formulierungen, Emulsionen, Suspensionen, Dispersionen und Puder werden, bezogen auf die fertigen Mittel, bevorzugt 0,01 bis 10 Gew.-%, besonders bevorzugt 0,1 bis 5 Gew.-%, insbesondere bevorzugt 0,5 bis 3 Gew.-%, an Copolymeren verwendet.

Die Copolymere können zusammen mit Hilfs- und Zusatzstoffe verwendet werden. Hilfs- und Zusatzstoffe sind anionische Tenside, kationische Tenside, nichtionische Tenside, zwitter-ionische Tenside und/oder amphotere Tenside, Ölkörper, Emulgatoren und Co-Emulgatoren, kationische Polymere, Filmbildner, sowie weitere gebräuchliche Zusätze, wie z.B. Überfettungsmittel, feuchtigkeitsspendende Mittel, Stabilisatoren, biogene Wirkstoffe, Glycerin, Konservierungsmittel, Perlglanzmittel, Farb- und Duftstoffe, Lösungsmittel, Hydrotrope, Trübungsmittel, weitere Verdickungsmittel und Dispergiermittel, ferner Eiweißderivate wie Gelatine, Collagenhydrolysate, Polypeptide auf natürlicher und synthetischer Basis, Eigelb, Lecithin, Lanolin und Lanolinderivate, Fettalkohole, Silicone, deodorierende Mittel, Stoffe mit keratolytischer und keratoplastischer Wirkung, Enzyme und Trägersubstanzen, Antioxidation, UV-Lichtschutzfilter, Pigmente und Metalloxide, sowie antimikrobiell wirkende Agentien enthalten.

In einer bevorzugten Ausführungsform werden die Copolymere, bezogen auf die fertigen Mittel, zusammen mit bevorzugt 2 bis 70 Gew.-%, besonders bevorzugt 5 bis 40 Gew.-%, insbesondere bevorzugt 12 bis 35 Gew.-% an Tensiden verwendet.

Als anionische Tenside eignen sich (C₁₀-C₂₀)-Alkyl- und Alkylencarboxylate, Alkylethercarboxylate, Fettalkoholsulfate, Fettalkoholethersulfate, Alkylamidsulfate und -sulfonate, Fettsäurealkylamidpolyglykolethersulfate, Alkansulfonate und Hydroxyalkansulfonate, Olefinsulfonate, Acylester von Isethionaten, α -Sulfofettsäureester, Alkylbenzolsulfonate, Alkylphenolglykolethersulfonate, Sulfosuccinate, Sulfobernsteinsäurehalbester und -diester, Fettalkoholetherphosphate, Eiweiß-Fettsäure-Kondensationsprodukte, Alkylmonoglyceridsulfate und -sulfonate, Alkylglyceridethersulfonate, Fettsäuremethyltauride, Fettsäuresarkosinate, Sulforicinoleate, Acylglutamate. Diese Verbindungen und deren Mischungen werden in Form ihrer wasserlöslichen oder in Wasser dispergierbaren Salze benutzt, beispielsweise der Natrium-, Kalium-, Magnesium-, Ammonium-, Mono-, Di- und Triethanolammonium- sowie analogen Alkylammonium-Salze.

Der Gewichtsanteil der anionischen Tenside beträgt bevorzugt 2 bis 30 Gew.-%, besonders bevorzugt 5 bis 25 Gew.-%, insbesondere bevorzugt 12 bis
22 Gew.-%, bezogen auf das fertige Mittel.
Geeignete kationische Tenside sind beispielsweise quartäre Ammonium-Salze wie Di-(C₁₀-C₂₄)-Alkyl-dimethylammoniumchlorid oder -bromid, vorzugsweise Di-(C₁₂-C₁₈)-Alkyl-dimethylammoniumchlorid oder-bromid; (C₁₀-C₂₄)-Alkyl-dimethylethylammoniumchlorid oder -bromid; (C₁₀-C₂₄)-Alkyl-trimethylammoniumchlorid oder -bromid, vorzugsweise Cetyltrimethylammoniumchlorid oder -bromid und (C₂₀-C₂₂)-Alkyl-trimethylammoniumchlorid oder -bromid; (C₁₀-C₂₄)-Alkyldimethylbenzyl-ammoniumchlorid oder -bromid, vorzugsweise (C₁₂-C₁₈)-Alkyldimethylbenzyl-ammoniumchlorid; N-(C₁₀-C₁₈)-Alkyl-pyridiniumchlorid oder-bromid, vorzugsweise N-(C₁₂-C₁₆)-Alkyl-pyridiniumchlorid oder -bromid; N-(C₁₀-C₁₈)-Alkyl-isochinolinium-chlorid, -bromid oder -monoalkylsulfat; N-(C₁₂-C₁₈)-Alkylpolyoylaminoformylmethyl-pyridiniumchlorid; N-(C₁₂-C₁₈)-Alkyl-N-methylmorpholinium-chlorid, -bromid oder -monoalkylsulfat; N-(C₁₂-C₁₈)-Alkyl-N-ethylmorpholinium-chlorid, -bromid oder -monoalkylsulfat; (C₁₆-C₁₈)-Alkyl-pentaoxethylammonium-chlorid; Diisobutyl-phenoxyethoxyethyldimethylbenzylammoniumchlorid; Salze des N,N-Diethylaminoethylstearylamids und -oleylamids mit Salzsäure, Essigsäure, Milchsäure, Zitronensäure, Phosphorsäure; N-Acylaminoethyl-N,N-diethyl-N-methyl-ammoniumchlorid, -bromid oder -monoalkylsulfat und N-Acylaminoethyl-N,N-diethyl-N-benzyl-ammonium-chlorid, -bromid oder -monoalkylsulfat, wobei Acyl vorzugsweise für Stearyl oder Oleyl steht.

Der Gewichtsanteil der kationischen Tenside (z.B. im Falle von hair conditioner) liegt bevorzugt im Bereich von 1 bis 10 Gew.-%, besonders bevorzugt 2 bis 7 Gew.-%, insbesondere besonders bevorzugt 3 bis 5 Gew.-%.

Als nichtionische Tenside eignen sich bevorzugt Fettalkoholethoxylate (Alkylpolyethylenglykole); Alkylphenolpolyethylenglykole; Alkylmercaptanpolyethylenglykole; Fettaminethoxylate (Alkylaminopolyethylenglykole); Fettsäureethoxylate (Acylpolyethylenglykole); Polypropylenglykolethoxylate (Pluronics^{®}; Fettsäurealkylolamide, (Fettsäureamidpolyethylenglykole); N-Alkyl-, N-Alkoxypolyhydroxyfettsäureamid, Saccharoseester; Sorbitester und der Polyglykolether.

Der Gewichtsanteil der nichtionischen Tenside (z.B. im Falle von Rinse-off-Produkten) liegt bevorzugt im Bereich von 1 bis 20 Gew.-%, besonders bevorzugt 2 bis 10 %, insbesondere bevorzugt 3 bis 7 Ges.-%.

Bevorzugte Amphotenside sind N-(C₁₂-C₁₈)-Alkyl-β-aminopropionate und N-(C₁₂-C₁₈)-Alkyl-β-iminodipropionate als Alkali- und Mono-, Di- und Trialkylammonium-Salze; N-Acylaminoalkyl-N,N-dimethyl-acetobetain, vorzugsweise N-(C₈-C₁₈)-Acylaminopropyl-N,N-dimethylacetobetain; (C₁₂-C₁₈)-Alkyl-dimethyl-sulfopropyl-betain; Amphotenside auf Basis Imidazolin (Handelsname: Miranol^{®}, Steinapon^{®}), vorzugsweise das Natrium-Salz des 1-(β-Carboxy-methyloxyethyl)-1-(carboxymethyl)-2-lauryl-imidazoliniums; Aminoxid, z.B. (C₁₂-C₁₈)-Alkyldimethylaminoxid, Fettsäureamidoalkyl-dimethylaminoxid.

Der Gewichtsanteil der amphoteren Tenside (z.B. im Falle von Rinse-off-Produkten) liegt bevorzugt im Bereich von 0,5 bis 20 Gew.-%, besonders bevorzugt 1 bis 10 Gew.-%.

Für die Verwendungen bevorzugte Tenside sind Laurylsulfat, Laurethsulfat, Cocoamidopropylbetain, Natriumcocoylglutamat, Lauroamphoacetat.

Des weiteren können die Copolymer zusammen mit schaumverstärkenden Co-Tensiden aus der Gruppe Alkylbetaine, Alkylamidobetaine, Aminopropionate, Aminoglycinate, Imidazoliniumbetaine und Sulfobetaine, Aminoxide und Fettsäurealkanolamide oder Polyhydroxyamide eingesetzt werden.

Unter Ölkörper ist jegliche Fettsubstanz zu verstehen, die bei Raumtemperatur (25°C) flüssig ist.
Die Fett-Phase kann ein oder mehrere Öle umfassen, die vorzugsweise aus folgenden Ölen ausgewählt werden:
Silikonöle, flüchtig oder nicht flüchtig, linear, verzweigt oder ringförmig, eventuell organisch modifiziert; Phenylsilikone, Silikonharze und -gummis, die bei Raumtemperatur fest oder flüssig sind; Mineralöle, wie Paraffin- oder Vaselinöl; Öle tierischen Ursprungs, wie Perhydrosqualen oder Lanolin; Öle pflanzlichen Ursprungs, wie flüssige Triglyceride, z.B. Sonnenblumen-, Mais-, Soja-, Reis-, Jojoba-, Babusscu-, Kürbis-, Traubenkern-, Sesam-, Walnuss-, Aprikosen-, Makadamia-, Avocado-, Süßmandel-, Wiesenschaumkraut-, Ricinusöl, Triglyceride der Capryl/Caprinsäuren, Olivenöl, Erdnussöl, Rapsöl und Kokosnussöl;
synthetische Öle, wie Purcellinöl, Isoparaffine, lineare und/oder verzweigte Fettalkohole und Fettsäureester, bevorzugt Guerbetalkohole mit 6 bis 18, vorzugsweise 8 bis 10, Kohlenstoffatomen; Ester von linearen (C₆-C₁₃)-Fettsäuren mit linearen (C₆-C₂₀)-Fettalkoholen; Ester von verzweigten (C₆-C₁₃)-Carbonsäuren mit linearen (C₆-C₂₀)-Fettalkoholen; Ester von linearen (C₆-C₁₈)-Fettsäuren mit verzweigten Alkoholen, insbesondere 2-Ethylhexanol; Ester von linearen und/oder verzweigten Fettsäuren mit mehrwertigen Alkoholen (wie z.B. Dimerdiol oder Trimerdiol) und/oder Guerbetalkoholen; Triglyceride auf Basis (C₆-C₁₀)-Fettsäuren; Ester, wie Dioctyladipat, Diisopropyl dimer dilineloat; Propylenglykoleldicaprilat oder Wachse wie Bienenwachs Paraffinwachs oder Mikrowachse, gegebenenfalls in Kombination mit hydrophilen Wachsen, wie z.B. Cetylstearylalkohol; fluorierte und perfluorierte Öle; fluorierte Silikonöle; Gemische der vorgenannten Substanzen.

Als nichtionogene Co-Emulgatoren kommen u.a. in Betracht Anlagerungsprodukte von 0 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe und an Sorbitan- bzw. Sorbitolester; (C₁₂-C₁₈)-Fettsäuremono- und -diester von Anlagerungsprodukten von 0 bis 30 Mol Ethylenoxid an Glycerin; Glycerinmono- und -diester und Sorbitanmono- und -diester von gesättigten und ungesättigten Fettsäuren mit 6 bis 22 Kohlenstoffatomen und ggfs. deren Ethylenoxidanlagerungsprodukten; Anlagerungsprodukte von 15 bis 60 Mol Ethylenoxid an Rizinusöl und/oder gehärtetes Rizinusöl; Polyol- und insbesondere Polyglycerinester, wie z.B. Polyglycerinpolyricinoleat und Polyglycerinpoly-12-hydroxystearat. Ebenfalls geeignet sind Gemische von Verbindungen aus mehreren dieser Substanzklassen.

Als ionogene Co-Emulgatoren eignen sich z.B. anionische Emulgatoren, wie mono-, di- oder tri-Phosphorsäureester, aber auch kationische Emulgatoren wie mono-, di- und tri-Alkylquats und deren polymere Derivate.

Als kationische Polymere eignen sich die unter der INCI-Bezeichnung "Polyquaternium" bekannten, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, sowie Polyquaternium 37&mineral oil&PPG trideceth (Salcare SC95), PVPdimethylaminoethylmethacrylat-Copolymer, Guar-hydroxypropyltriammoniumchloride, sowie Calciumalginat und Ammoniumalginat. Des weiteren können eingesetzt werden kationische Cellulosederivate; kationische Stärke; Copolymere von Diallylammoniumsalzen und Acrylamiden; quaternierte Vinylpyrrolidonl Vinylimidazol-Polymere; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan.

Geeignete Siliconverbindungen sind beispielsweise Dimethylpolysiloxan, Methylphenylpolysiloxane, cyclische Silicone und amino-, fettsäure-, alkohol-, polyether-, epoyx-, fluor- und/oder alkylmodifizierte Siliconverbindungen, sowie Polyalkylsiloxane, Polyalkylarylsiloxane, Polyethersiloxan-Copolymere, wie in US 5104 645 und den darin zitierten Schriften beschrieben, die bei Raumtemperatur sowohl flüssig als auch harzförmig vorliegen können.

Geeignete Filmbildner sind, je nach Anwendungszweck wasserlösliche Polyurethane, beispielsweise C10-Polycarbamylpolyglycerylester, Polyvinylalkohol, Polyvinylpyrrolidon, -copolymere, beispielsweise Vinylpyrrolidon/Vinylacetatcopolymer, wasserlösliche Acrylsäurepolymerel Copolymere bzw. deren Ester oder Salze, beispielsweise Partialestercopolymere der Acryl/ Methacrylsäure und Polyethylenglykolether von Fettalkoholen, wie Acrylat/Steareth-20-Methacrylat Copolymer, wasserlösliche Cellulose, beispielsweise Hydroxymethylcellulose, Hydroxyethylcellulose, Hydroxypropylcellulose, wasserlösliche Quaterniums, Polyquaterniums, Carboxyvinyl-Polymere, wie Carbomere und deren Salze, Polysaccharide, beispielsweise Polydextrose und Glucan.

Als Überfettungsmittel können Substanzen wie beispielsweise polyethoxylierte Lanolinderivate, Lecithinderivate, Polyolfettsäureester, Monoglyceride und Fettsäurealkanolamide verwendet werden, wobei die letzteren gleichzeitig als Schaumstabilisatoren dienen. Als feuchtigkeitsspendende Substanz stehen beispielsweise Isopropylpalmitat, Glycerin und/oder Sorbitol zu Verfügung.

Als Stabilisatoren können Metallsalze von Fettsäuren, wie z.B. Magnesium-, Aluminium- und/oder Zinkstearat eingesetzt werden. Unter biogenen Wirkstoffen sind beispielsweise Pflanzenextrakte und Vitaminkomplexe zu verstehen.

Zusätzlich können bei den Verwendungen organische Lösungsmittel mitverwendet werden. Prinzipiell kommen als organische Lösungsmittel alle ein- oder mehrwertigen Alkohole in Betracht. Bevorzugt werden Alkohole mit 1 bis 4 Kohlenstoffatomen wie Ethanol, Propanol, Isopropanol, n-Butanol, i-Butanol, t-Butanol, Glycerin und Mischungen aus den genannten Alkoholen eingesetzt. Weitere bevorzugte Alkohole sind Polyethylenglykole mit einer relativen Molekülmasse unter 2000. Insbesondere ist ein Einsatz von Polyethylenglykol mit einer relativen Molekülmasse zwischen 200 und 600 und in Mengen bis zu 45 Gew.-% und von Polyethylenglykol mit einer relativen Molekülmasse zwischen 400 und 600 in Mengen von 5 bis 25 Gew.-% bevorzugt. Weitere geeignete Lösungsmittel sind beispielsweise Triacetin (Glycerintriacetat) und 1-Methoxy-2-propanol.
Die Verwendung kann zusammen mit Ceramiden, Pseudoceramiden, FettsäureN-alkylpolyhydroxyalkylamiden, Cholesterin, Cholesterinfettsäureestern, Fettsäuren, Triglyceriden, Cerebrosiden, Phospholipiden und ähnlichen Stoffen als Pflegezusätze erfolgen.

Als UV-Filter eignen sich z.B. 4-Aminobenzoesäure;
3-(4'-Trimethylammonium)benzyliden-boran-2-on-methylsulfat;
3,3,5-Trimethyl-cyclohexylsalicylat; 2-Hydroxy-4-methoxybenzophenon;
2-Phenylbenzimidazol-5-sulfonsäure und ihre Kalium-, Natrium- und Triethanolaminsalze; 3,3'-(1,4-Phenylendimethin)-bis-(7,7-dimethyl-2-oxobicyclo[2.2.1]-heptan-1-methansulfonsäure und ihre Salze; 1-(4-tert.-Butylphenyl)-3-(4-methoxyphenyl)propan-1,3-dion, 3-(4'-Sulfo)-benzyliden-bornan-2-on und seine Salze; 2-Cyan-3,3-diphenyl-acrylsäure-(2-ethylhexylester);
Polymer von N-[2(und 4)-(2-oxoborn-3-ylidenmethyl)benzyl]-acrylamid; 4-Methoxyzimtsäure-2-ethyl-hexylester; ethoxyliertes Ethyl-4-amino-benzoat; 4-Methoxyzimtsäure-isoamylester; 2,4,6-Tris-[p-(2-ethylhexyloxycarbonyl)anilino]-1,3,5-triazin; 2-(2H-benzotriazol-2-yl)-4-methyl-6-(2-methyl-3-(1,3,3,3-tetramethyl-1-(trimethylsilyloxy)-disiloxanyl)propyl)phenol; 4,4'-[(6-[4-((1,1-dimethylethyl)-aminocarbonyl)phenylamino]-1,3,5-triazin-2,4-yl)diimino]bis-(benzoesäure-2-ethylhexylester); 3-(4'-Methylbenzyliden)-D,L-Campher; 3-Benzyliden-Campher; Salicylsäure-2-ethylhexylester; 4-Dimethylaminobenzoesäure-2-ethylhexylester; Hydroxy-4-methoxy-benzophenon-5-sulfonsäure (Sulisobenzonum) und das Natriumsalz; und/oder 4-Isopropylbenzylsalicylat.

Als Pigmente/Mikropigmente können Z.B. mikrofeines Titandioxid, Glimmer-Titanoxid, Eisenoxide, Glimmer-Eisenoxid, Zinkoxid, Siliziumoxide, Ultramarinblau, Chromoxide, eingesetzt werden.

Als Antioxidantien eignen sich beispielsweise Superoxid-Dismutase, Tocopherol (Vitamin E) und Ascorbinsäure (Vitamin C).

Als Konservierungsmittel in Betracht kommen beispielsweise Phenoxyethanol, Parabene, Pentandiol oder Sorbinsäüre.
Als Farbstoffe können die für kosmetische Zwecke geeigneten und zugelassenen Substanzen verwendet werden.
Als antifungizide Wirkstoffe eignen sich bevorzugt Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine, Zn-Pyrethion und Oczopyrox.

Um die rheologischen Eigenschaften von wässrigen oder lösungsmittelhaltigen Emulsionen oder Suspensionen einzustellen, werden in der Fachliteratur eine Vielzahl von unterschiedlichen Systemen angegeben. Bekannt sind beispielsweise Celluloseether und andere Cellulosederivate (z.B. Carboxymethylcellulose, Hydroxyethylcellulose), Gelatine, Stärke und Stärkederivate, Natriumalginate, Fettsäurepolyethylenglykolester, Agar-Agar, Traganth oder Dextrine. Als synthetische Polymere kommen verschiedene Materialien zum Einsatz, wie z.B. Polyvinylalkohole, Polyacrylamide, Polyvinylamide, Polysulfonsäuren, Polyacrylsäure, Polyacrylsäureester, Polyvinylpyrrolidon, Polyvinylmethylether, Polyethylenoxide, Copolymere aus Maleinsäureanhydrid und Vinylmethylether, sowie diverse Mischungen und Copolymerisate aus den o.a. Verbindungen, einschließlich ihrer verschiedenen Salze und Ester. Diese Polymere können wahlweise vernetzt oder unvernetzt sein.

Wesentlich für die Erfindung ist, dass die beschriebenen Acryloyldimethyltaurinsäure-enthaltenden Copolymere auch ohne Mitverwendung eines zusätzlichen Co-Emulgators und/oder ohne Mitverwendung eines zusätzlichen Konsistenzgebers eingesetzt werden können. Die Mitverwendung von Co-Emulgatoren und/oder Konsistenzgebern ist daher nicht zwingend, aber möglich. Eine Kombination mit anderen bekannten Co-Emulgatoren und/oder Konsistenzgebern kann zur Einstellung spezieller kosmetischer Profile und zur Ausnutzung synergistischer Effekte wünschenswert sein.

Die erzielte Beschaffenheit ist ausgesprochen vorteilhaft:
die Emulsionen sind cremig und salbig und haben überhaupt nicht das gelartige oder sogar gelatineartige Aussehen gewisser Emulsionen nach dem Stand der Technik, bei denen die äußere wässrige Phase verdickt ist.
Auch das kosmetische Gefühl auf der Haut ist sehr gut:
beim Auftragen verleiht die Emulsion ein Gefühl der Frische und des Komforts, wobei sie gleichzeitig gehaltvoll und nährend wirkt; sie ist weich und komfortabel und in keiner Weise klebrig.

Bei den erfindungsgemäßen Verwendungen besitzen die Mittel üblicherweise einen pH Wert im Bereich 2 bis 12, bevorzugt pH 3 bis 8.

Die nachfolgenden Beispiele und Anwendungen sollen die Erfindung näher erläutern, ohne sie jedoch darauf zu beschränken (bei allen Prozentangaben handelt es sich um Gew.-%). Bei den in den Beispielen verwendeten Copolymeren handelt es sich um Vertreter der in der Beschreibung bereits aufgeführten besonders bevorzugten Copolymere Nr.1 bis Nr.67. Die Herstellung erfolgte nach den dort angegebenen Verfahren 1, 2, 3 oder 4 unter Verwendung der bevorzugten Initiatoren und Lösemittel.

### Beispiel 1: O/W - Hautmilch

### Zusammensetzung

| | | |
|---|---|---|
| A | Copolymer Nr. 21 | 0,50 % |
| | Isopropylpalmitat | 4,00 % |
| | Mandelöl 5,00 % | 4,00 % |
| | Weizenkeimöl | 1,00 % |
| | ^{®}Cetiol SN (Henkel) | 8,00 % |
| | Cetearylisononanoat | |
| B | ^{®}Aristoflex AVC (Clariant) | 0,30 % |
| | Ammonium Acryloyldimethyltaurate/VP | Copolymer |
| C | Wasser | ad 100% |
| D | Duftstoffe | 0,30 % |

### Herstellung

| | |
|---|---|
| I | A und B mischen, dann C hinzugeben |
| II | D zu 1 hinzurühren |
| III | Emulsion homogenisieren |

Copolymer Nr. 21 wird als Emulgator eingesetzt und erhöht zudem die Konsistenz.

### Beispiel 2: O/W After-Sun-Milch

### Zusammensetzung:

| | | |
|---|---|---|
| A | Copolymer Nr. 34 | 1,00 % |
| | Isopropylpalmitat | 5,00 % |
| | ^{®}Cetiol SN (Henkel) | 4,00 % |
| | Cetearylisononanoat | |
| | Sojaöl | 4,00 % |
| | ^{®}Miglyol 812 (Dynamit Nobel) | 3,00 % |
| | Capryl/Caprin Triglyceride | |
| | Jojobaöl | 3,00 % |
| | Weizenkeimöl | 1,00 % |
| D | ^{®}AQUAMOLLIN BC Plv. hochkonz. (Clariant) | 0,10 % |
| | Ethylendiamine Tetraacetic Acid Sodium Salt | |
| | Citronensäure (10 %ig) | 0,30 % |
| | Wasser | 68,80 % |
| | Glycerin | 3,00 % |
| | ALLANTOIN (Clariant) | 0,20 % |
| | Allantoin | |
| | Konservierungsmittel | q.s. |
| E | Ethanol | 1,50 % |
| | Parfümöl | 0,30 % |

### Herstellung

| | |
|---|---|
| I | Die Komponenten von A homogen verrühren |
| II | Bei ca. 35°C D in I einrühren. |
| III | Die Emulsion homogenisieren. |

Copolymer Nr. 34 dient als Emulgator und Verdicker. Zusätzlich wird ein samtiges, weiches Hautgefühl erzeugt.

### Beispiel 3: W/O Creme

### Zusammensetzung

| | | |
|---|---|---|
| A | ^{®}HOSTACERIN DGI (Clariant) | 4,00 % |
| | Polyglyceryl-2 Sesquiisostearate | |
| | Beeswax | 2,00 % |
| | Copolymer Nr. 10 | 1,5 % |
| | Mineral Oil, low viscosity | 5,00 % |
| | Vaseline | 10,00 % |
| | ^{®}Cetiol V (Henkel KGaA) | 5,00 % |
| | Decyl Oleat | |
| B | 1,2-Propylene glycol | 3,00 % |
| | Wasser | 69,10 % |
| | Preservative | q.s. |
| C | Fragrance | 0,40 % |

### Herstellung

| | |
|---|---|
| I | A bei 80°C aufschmelzen |
| II | B auf 80°C erhitzen. |
| III | II in I einrühren. |
| IV | Stir until cool. |
| V | At 35°C add C to IV. |

Copolymer Nr. 10 wird als Konsistenzgeber und Stabilisator eingesetzt. Beispiele für Tensid Formulierungen

### Beispiel 4: body wash

### Zusammensetzung

| | | |
|---|---|---|
| A | ^{®}GENAPOL LRO liquid (Clariant) | 40,00 % |
| | Sodium Laureth sulfate | |
| B | Fragrance | 0,30 % |
| C | Wasser | 52,70 % |
| | Dyestuff | q.s. |
| | Preservative | q.s. |
| | ^{®}GENAGEN LDA (Clariant) | 6,00 % |
| | Disodium Lauroamphodiacetate | |
| | Citric Acid | q.s. |
| D | Copolymer It. Bsp.1 | 1,00 % |

### Herstellung

| | |
|---|---|
| I | B in A einrühren |
| II | Komponenten aus C nacheinander zu I zugeben |
| III | pH auf 5,5 einstellen |
| IV | Einstellen der Viskosität durch Einrühren von D in II |

Polymer lt. Beispiel 1 wird als Verdicker eingesetzt. In Kombination mit den genannten Tensiden wird Viskosität erhöht.

### Zusammensetzung

| | | |
|---|---|---|
| A | Wasser | 60,70 % |
| | ^{®}GENAPOL ZRO liquid (Clariant) | 25,00 % |
| | Sodium Laureth Sulfate | |
| | ^{®}HOSTAPON CLG (Clariant) | 8,00 % |
| | Sodium Lauroyl Glutamate | |
| | ^{®}GENAPOL SBE (Clariant) | 5,00 % |
| | Disodium Laureth Sulfosuccinate | |
| | Fragrance | 0,30 % |
| | Dyestuff solution | q.s. |
| | Preservative | q.s. |
| B | Polymer Nr. 44 | 1,00 % |

### Herstellung

| | |
|---|---|
| I | B in A lösen |
| II | gegebenenfalls pH einstellen |

Polymer Nr. 44 dient als Konditioniermittel und erhöht in Kombination mit den Tensiden die Viskosität der Formulierung.

### Beispiel 6: Antischuppen Shampoo, klar

### Zusammensetzung

| | | | |
|---|---|---|---|
| A | ^{®}OCTOPIROX | (Clariant) | 0,50 % |
| | Piroctone Olamine | | |
| B | Wasser | | 10,00 % |
| C | ^{®}GENAPOL LRO LIQUID | (Clariant) | 30,00% |
| | Sodium Laureth Sulfate | | |
| D | ^{®}Belsil DMC 6032 | (Wacker Chemie) | 1,50 % |
| | Dimethicone Copolyol Acetate | | |
| | Fragrance | | 0,30 % |
| E | ^{®}ALLANTOIN | (Clariant) | 0,30 % |
| F | Wasser | | 46,40 % |
| G | Dyestuff solution | | q.s. |
| | Panthenol | (Hoffmann La Roche) | 1,00 % |
| | ^{®}GENAGEN CAB | (Clariant) | 8,00 % |
| | Cocamidopropyl Betaine | | |
| H | Polymer It. Beispiel 28 | | 1,10 % |

### Herstellung

| | |
|---|---|
| I | A mit B mischen |
| II | C in I einrühren bis klare Lösung |
| III | Komponenten aus D nacheinander in I geben |
| IV | E in F unter Erwärmen einrühren und dann in I einrühren |
| V | Komponenten aus G nacheinander in I geben |
| VI | gegebenenfalls pH einstellen |
| VII | Einstellen der Viskosität durch Einrühren von H in I |

Polymer It. Beispiel 28 dient in diesem Beispiel einerseits als Konsistenzgeber und insbesondere als Stabilisator und Dispergiermittel für die unlöslichen Ölkomponenten. Zudem hinterlässt Polymer It. Beispiel 28 einen konditionierenden Effekt auf dem Haar.

### Beispiel 7: Antischuppen Shampoo, perlglänzend

### Zusammensetzung

| | | | |
|---|---|---|---|
| A | Wasser | | 38,7 % |
| B | ^{®}HOSTAPON SCI-65 | (Clariant) | 3,00 % |
| | Sodium Cocoyl Isethionate | | |
| C | ^{®}GENAPOL LRO liquid | (Clariant) | 35,00 % |
| | Sodium Laureth Sulfate | | |
| | ^{®}HOSTAPON KCG | (Clariant) | 5,00 % |
| | Sodium Cocoyl Glucamate | | |
| | ^{®}Belsil DMC 6032 | (Wacker) | 1,00 % |
| | Dimethicone Copolyol Acetate | | |
| | Fragrance | | 0,30 % |
| | ^{®}GENAGEN CAB | (Clariant) | 9,00 % |
| | ^{®}Cocamidopropyl Betaine | | |
| | GENAPOL TSM | (Clariant) | 4,00 % |
| | PEG-3 Distearate (and) Sodium Laureth Sulfate | | |
| | Merquat 550 | | 0,50 % |
| | Polyquaternium-7 | | |
| | Zinc Omadine FPS (Olin) | | |
| | Zinc Pyrithione (48 %ig) | | 2,50 % |
| | Copolymer Nr. 14 | | 1,00 % |
| | Dyestuff solution | | q.s. |
| | Preservative | | q.s. |

### Herstellung

| | |
|---|---|
| I | Bei 80°C B in A lösen |
| II | Nach Abkühlen auf ca. 35°C, Komponenten C nacheinander zuzugeben |

Polymer It. Beispiel 14 dient in diesem Beispiel einerseits als Konsistenzgeber (in Kombination mit den Tensiden) und insbesondere als Stabilisator und
Dispergiermittel für die unlöslichen Feststoffe.

Beispiele für Gele

### Beispiel 8: Haargel mit konditionierenden Eigenschaften

### Zusammensetzung

| | | |
|---|---|---|
| A | Wasser | 92,00 % |
| | Panthenol | 1,50 % |
| | UVAsorb S5 | 0,05 % |
| | Benzophenone-4 | |
| | Dyestuff solution | q.s. |
| | Preservative | q.s. |
| B | ^{®}Emulsogen HCO 040 (Clariant) | 0,50 % |
| | PEG-40 Hydrogenated Castor Oil | |
| | parfume | q.s. |
| C | Copolymer Nr. 26 | 2,00 % |
| D | Gafquat 755N (ISP) | 2,50 % |
| | Polyquaternium-11 | |

### Herstellung

| | |
|---|---|
| I | Komponenten A mischen |
| II | Komponenten B mischen und zu I geben |
| III | C zu D zu I geben |

Copolymer Nr. 26 wirkt als effizienter Verdicker mit zusätzlich konditionierenden Eigenschaften.

### Beispiel 9: Haargel mit starkem Halt

### Zusammensetzung

| | | |
|---|---|---|
| A | Wasser | 71,50 % |
| | PVP K-30 (ISP) | 4,00 |
| | PVP | |
| | Ethanol | 30,00 % |
| | Panthenol | 0,50 % |
| | UVAsorb S5 | 0,05 % |
| | Benzophenone-4 | |
| | Dyestuff solution | q.s. |
| | Preservative | q.s. |
| B | Abil B 88.51 (Goldschmidt) | 1,00 % |
| | Dimethicone Copolyol | |
| | ^{®}Emulsogen HCO 040 (Clariant) | 0,50 % |
| | PEG-40 Hydrogenated Castor Oil | |
| | parfume | q.s. |
| C | Copolymer Nr. 63 | 2,50 % |

### Herstellung

| | |
|---|---|
| I | Komponenten A mischen |
| II | Komponenten B in I geben |
| III | Komponenten C in I geben |

Copolymer Nr. 63 wird als Verdicker mit sehr guter Alkoholtoleranz verwendet und dient zudem als Suspendiermittel und Stabilisator für die unlöslichen Ölanteile.

## Patentansprüche

1. Verwendung von Copolymeren, erhältlich durch radikalische Copolymerisation von
A) Acryloyldimethyltaurinsäure und/oder Acryloyldimethyltauraten,
B) gegebenenfalls einem oder mehreren weiteren olefinisch ungesättigten, nicht kationischen, gegebenenfalls vernetzenden, Comonomeren, die wenigstens ein Sauerstoff-, Stickstoff-, Schwefel- oder Phosphoratom aufweisen und ein Molekulargewicht kleiner 500 g/mol besitzen,
C) gegebenenfalls einem oder mehreren olefinisch ungesättigten, kationischen Comonomeren, die wenigstens ein Sauerstoff-, Stickstoff-, Schwefel- oder Phosphoratom aufweisen und ein Molekulargewicht kleiner 500 g/mol besitzen,
D) gegebenenfalls einer oder mehreren mindestens monofunktionellen, zur radikalischen Polymerisation befähigten, siliziumhaltigen Komponente(n),
E) gegebenenfalls einer oder mehreren mindestens monofunktionellen, zur radikalischen Polymerisation befähigten, fluorhaltigen Komponente(n),
F) einem oder mehreren einfach oder mehrfach olefinisch ungesättigten, gegebenenfalls vernetzenden, Makromonomeren, die jeweils mindestens ein Sauerstoff-, Stickstoff-, Schwefel- oder Phosphoratom besitzen und ein zahlenmittleres Molekulargewicht größer oder gleich 200 g/mol aufweisen, wobei es sich bei den Makromonomeren nicht um eine siliziumhaltige Komponente D) oder fluorhaltige Komponente E) handelt, und die Makromonomere acrylisch- oder methacrylisch monofunktionalisierte Alkylethoxylate gemäß Formel (IV) sind 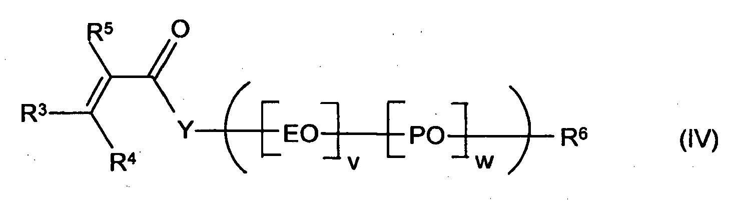 worin
R₃ und R₄ gleich H oder -CH₃ sind, R₅ gleich H oder -CH₃ ist; und R₆ gleich einem n-aliphatischen, iso-aliphatischen, olefinischen, cycloaliphatischen, arylaliphatischen oder aromatischen (C₁-C₃₀)-Kohlenwasserstoffrest ist, v und w die stöchiometrischen Koeffizienten betreffend die Ethylenoxideinheiten (EO) und Propylenoxideinheiten (PO) sind, wobei
v und w unabhängig voneinander 0 bis 500 betragen, die Summe aus v und w im Mittel ≥ 1 sein muss und die Verteilung der EO- und PO-Einheiten über die Makromonomerkette statistisch, blockartig, alternierend oder gradientenartig sein kann und Y -O-, -C(O)-, -C(O)-O-, -5-, -O-CH₂-CH(O-)-CH₂OH, -O-CH₂-CH(OH)-CH₂O-, -O-SO₂-O-, -O-SO-O-, -PH-, -P(CH₃)-, -P0₃-, -NH- oder -N(CH₃)- ist,
G) wobei die Copolymerisation gegebenenfalls in Gegenwart mindestens eines polymeren Additivs mit zahlenmittleren Molekulargewichten von 200 g/mol bis 10⁹ g/mol erfolgt,
als Verdicker, Dispergiermittel, Suspendiermittel, Emulgator, Stabilisator, Solubilisator, Konditionierer, Konsistenzgeber, Gleitmittel, Haftmittel und/oder Conditioner in kosmetischen, pharmazeutischen und dermatologischen Mitteln.

2. Verwendung nach Anspruch 1, **dadurch gekennzeichnet, dass** es sich bei den Comonomeren B) um ungesättigte Carbonsäuren, Salze ungesättigter Carbonsäuren, Anhydride ungesättigter Carbonsäuren, Ester ungesättigter Carbonsäuren mit aliphatischen, olefinischen, cycloaliphatischen, arylaliphatischen oder aromatischen Alkoholen mit 1 bis 22 C-Atomen, offenkettige N-Vinylamide, cyclische N-Vinylamide mit einer Ringgröße von 3 bis 9, Amide der Acrylsäure, Amide der Methacrylsäure, Amide substituierter Acrylsäuren, Amide substituierter Methacrylsäuren, 2-Vinylpyridin, 4-Vinylpyridin, Vinylacetat; Styrol, Acrylnitril, Vinylchlorid, Vinylidenchlorid, Tetrafluorethylen, Vinylphosphonsäure oder deren Ester oder Salze, Vinylsulfonsäure oder deren Ester oder Salze, Allylphosphonsäure oder deren Ester oder Salze und/oder Methallylsulfonsäure oder deren Ester oder Salze handelt.

3. Verwendung nach Anspruch 1 und/oder 2, **dadurch gekennzeichnet, dass** es sich beiden Comonomeren C) Diallyldimethylammoniumchlorid (DADMAC), [2-(Methacryloyloxy)ethyl]trimethylammoniumchlorid (MAPTAC), [2-(Acryloyloxy)ethyl]trimethylammoniumchlorid, [2-Methacrylamidoethyl]trimethylammoniumchlorid, [2-(Acrylamido)ethyl]trimethylammoniumchlorid, N-Methyl-2-vinylpyridiniumchlorid N-Methyl-4-vinylpyridiniumchlorid Dimethylaminoethylmethacrylat, Dimethylaminopropylmethacrylamid, Methacryloylethyl-N-oxid und/oder Methacryloylethyl-betain handelt.

4. Verwendung nach mindestens einem der Ansprüche 1 bis 3, **dadurch gekennzeichnet, dass** es sich bei den siliziumhaltigen Komponenten D) um Verbindungen der Formel (I)
R¹-Z-[(Si(R³R⁴)-O-)_{w}-(Si(R⁵R⁶)-O)ₓ-]- R² (I)
handelt, wobei
R¹ einen Vinyl-, Allyl-, Methallyl-, Methylvinyl-, Acryl-, Methacryl-, Crotonyl-, Senecionyl-, Itaconyl-, Maleinyl-, Fumaryl- oder ein Styrylrest darstellt;
Z eine chemische Brücke, bevorzugt ausgewählt aus -O-, -((C₁ - C₅₀) Alkylen)-, -((C₆ - C₃₀) Arylen)-, -((C₅ - C₈) Cycloalkylen)-, -((C₁-C₅₀) Alkenylen)-, -(Polypropylenoxid)ₙ-, -(Polyethylenoxid)ₒ-, -(Polypropylenoxid)ₙ(Polyethylenoxid)ₒ-, wobei n und o unabhängig voneinander Zahlen von 0 bis 200 bedeuten und die Verteilung der EO/PO-Einheiten statistisch oder blockförmig sein kann, -((C₁ - C₁₀) Alkyl)-(Si(OCH₃)₂)- und -(Si(OCH₃)₂)-, darstellt;
R³, R⁴, R⁵ und R⁶ unabhängig voneinander -CH₃, -O-CH₃, -C₆H₅ oder -O-C₆H₅ bedeuten;
w, x Zahlen von 0 bis 500 bedeuten, wobei entweder w oder x größer Null sein muss, und
R² einen gesättigten oder ungesättigten, aliphatischen cycloaliphatischen, arylaliphatischen oder aromatischen Rest mit jeweils 1 bis 50 C-Atomen oder eine Gruppe der Formeln -OH, -NH₂, -N(CH₃)₂, -R⁷ oder eine Gruppe -Z-R¹ bedeutet, wobei Z und R¹ die obengenannten Bedeutungen haben und
R⁷ eine Gruppe der Formel -O-Si(CH₃)₃, -O-Si(Phenyl)₃, -O-Si(O-Si(CH₃)₃)₂CH₃) und -O-Si(O-Si(Ph)₃)₂Ph) bedeutet.

5. Verwendung nach mindestens einem der Ansprüche 1 bis 4, **dadurch gekennzeichnet, dass** es sich bei den fluorhaltigen Komponenten E) um Verbindungen der Formel (II)
R¹-Y-CᵣH₂ᵣCₛF₂ₛCF₃ (II)
handelt, wobei
R¹ eine polymerisationsfähige Funktion aus der Gruppe der vinylisch ungesättigten Verbindungen, bevorzugt einen Vinyl-, Allyl-, Methallyl-, Methylvinyl-, Acryl-, Methacryl-, Crotonyl-, Senecionyl-, Itaconyl-, Maleinyl-, Fumaryl- oder Styrylrest, darstellt;
Y eine chemische Brücke, bevorzugt -O-, -C(O)-, -C(O)-O-, -S-, -O-CH₂-CH(O-)-CH₂OH, -O-CH₂-CH(OH)-CH₂-O-, -O-SO₂-O- , -O-S(O)-O-, -PH-, -P(CH₃)-, -PO₃-, -NH-, -N(CH₃)-, -O-(C₁-C₅₀)Alkyl-O-, -O-Phenyl-O-, -O-Benzyl-O-, -O-(C₅-C₈)Cycloalkyl-O-, -O-(C₁-C₅₀)Alkenyl-O-, -O-(CH(CH₃)-CH₂-O)ₙ-, -O-(CH₂-CH₂-O)ₙ- und =O-([CH-CH₂-O]ₙ-[CH₂-CH₂-O]ₘ)ₒ-, wobei n, m und o unabhängig voneinander Zahlen von 0 bis 200 bedeuten, darstellt und
r,s stöchiometrische Koeffizienten darstellen, die unabhängig voneinander Zahlen zwischen 0 und 200 sind.

6. Verwendung nach mindestens einem der Ansprüche 1 bis 5, **dadurch gekennzeichnet, dass** es sich bei den polymeren Additiven G) um Homo- oder Copolymere aus N-Vinylformamid, N-Vinylacetamid, N-Vinylpyrrolidon, Ethylenoxid, Propylenoxid, Acryloyldimethyltaurinsäure, N-Vinylcaprolactam, N-Vinylmethylacetamid, Acrylamid, Acrylsäure, Methacrylsäure, N-Vinylmorpholid, Hydroxymethylmethacrylat, Diallyldimethylammoniumchlorid (DADMAC) und/oder [2-(Methacryloyloxy)ethyl]trimethylammoniumchlorid (MAPTAC); Polyalkylenglykole und/oder Alkylpolyglykole handelt.

7. Verwendung nach mindestens einem der Ansprüche 1 bis 6, **dadurch gekennzeichnet, dass** die Copolymerisation in Gegenwart mindestens eines polymeren Additivs G) erfolgt.

8. Verwendung nach mindestens einem der Ansprüche 1 bis 7, **dadurch gekennzeichnet, dass** die Copolymere vernetzt sind.

9. Verwendung nach mindestens einem der Ansprüche 1 bis 8, **dadurch gekennzeichnet, dass** die Copolymere durch Fällungspolymerisation in tert.-Butanol hergestellt werden.

10. Verwendung nach mindestens einem der Ansprüche 1 bis 9, **dadurch gekennzeichnet, dass**, bezogen auf die fertigen Mittel, 0,01 bis 10 Gew.-% der Copolymere verwendet werden.

11. Verwendung nach mindestens einem der Ansprüche 1 bis 10, **dadurch gekennzeichnet, dass** die Copolymere wasserlöslich oder wasserquellbar sind.

## Claims

1. Use of copolymers obtainable by free-radical copolymerization of
A) acryloyldimethyltaurine and/or acryloyldimethyltaurates,
B) if desired, one or more further olefinically unsaturated, noncationic, optionally crosslinking comonomers which have at least one oxygen, nitrogen, sulfur or phosphorus atom and possess a molecular weight of less than 500 g/mol,
C) if desired, one or more olefinically unsaturated, cationic comonomers which have at least one oxygen, nitrogen, sulfur or phosphorus atom and possess a molecular weight of less than 500 g/mol,
D) if desired, one or more silicon-containing components capable of free-radical polymerization and having a functionality of at least one,
E) if desired, one or more fluorine-containing components capable of free-radical polymerization and having a functionality of at least one,
F) one or more olefinically mono- or polyunsaturated, optionally crosslinking macromonomers each possessing at least one oxygen, nitrogen, sulfur or phosphorus atom and having a number-average molecular weight of greater than or equal to 200 g/mol, the macromonomers not being a silicon-containing component D) or fluorine-containing component E), and the macromonomers being acrylically or methacrylically monofunctionalized alkyl ethoxylates of formula (IV) 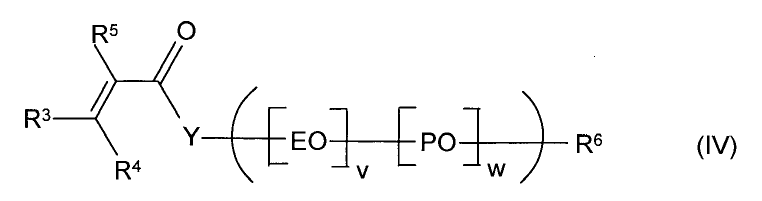 in which
R³ and R⁴ are H or -CH₃, R⁵ is H or -CH₃, and R⁶ is an n-aliphatic, iso-aliphatic, olefinic, cycloaliphatic, arylaliphatic or aromatic (C₁-C₃₀) hydrocarbon radical,
v and w are the stoichiometric coefficients relating to the ethylene oxide units (EO) and propylene oxide units (PO), and v and w amount independently of one another to from 0 to 500, it being necessary for the sum of v and w to be on average ≥ 1, and the distribution of the EO and PO units over the macromonomer chain can be random, blocklike, alternating or gradientlike, and Y is - O-, -C(O)-, -C(O)-O-, -S-, -O-CH₂-CH(O-)-CH₂OH, -O-CH₂-CH(OH)-CH₂-O-, -O-SO₂-O- , -O-S(O)-O-, -PH-, -P(CH₃)-, -PO₃-, -NH- or -N(CH₃)-,
G) the copolymerization taking place if desired in the presence of at least one polymeric additive having number-average molecular weights of from 200 g/mol to 10⁹ g/mol,
as thickeners, dispersants, suspension media, emulsifiers, stabilizers, solubilizers, conditioning agents, bodying agents, lubricants, adhesives and/or conditioners in cosmetic, pharmaceutical, and dermatological compositions.

2. Use according to Claim 1, **characterized in that** the comonomers B) are unsaturated carboxylic acids, salts of unsaturated carboxylic acids, anhydrides of unsaturated carboxylic acids, esters of unsaturated carboxylic acids with aliphatic, olefinic, cycloaliphatic, arylaliphatic or aromatic alcohols having 1 to 22 carbon atoms, open-chain N-vinyl amides, cyclic N-vinyl amides having a ring size of from 3 to 9, amides of acrylic acid, amides of methacrylic acid, amides of substituted acrylic acids, amides of substituted methacrylic acids, 2-vinylpyridine, 4-vinylpyridine, vinyl acetate; styrene, acrylonitrile, vinyl chloride, vinylidene chloride, tetrafluoroethylene, vinylphosphonic acid or the esters or salts thereof, vinylsulfonic acid or the esters or salts thereof, allylphosphonic acid or the esters or salts thereof and/or methallylsulfonic acid or the esters or salts thereof.

3. Use according to Claim 1 and/or 2, **characterized in that** the comonomers C) are diallyldimethylammonium chloride (DADMAC), [2-(methacryloyloxy)ethyl]trimethylammonium chloride (MAPTAC), [2-(acryloyloxy)ethyl]trimethylammonium chloride, [2-methacrylamidoethyl]trimethylammonium chloride, [2-(acrylamido)ethyl]trimethylammonium chloride, N-methyl-2-vinylpyridinium chloride, N-methyl-4-vinylpyridinium chloride, dimethylaminoethyl methacrylate, dimethylaminopropylmethacrylamide, methacryloylethyl N-oxide and/or methacryloylethylbetaine.

4. Use according to at least one of Claims 1 to 3, **characterized in that** the silicon-containing components D) are compounds of the formula (I)
R¹ - Z- [(Si(R³R⁴)-O-)_{w}-(Si(R⁵R⁶)-O)ₓ-]- R² (I)
where
R¹ represents a vinyl, allyl, methallyl, methylvinyl, acryloyl, methacryloyl, crotonyl, senecionyl, itaconyl, maleyl, fumaryl or styryl radical;
Z is a chemical bridge, preferably selected from -O-, - ((C₁-C₅₀) alkylene) -, - ((C₆-C₃₀) arylene) -, - (C₅-C₈) cycloalkylene) -, - ((C₁-C₅₀) alkenylene) -, -(polypropylene oxide)ₙ-, -(polyethylene oxide)ₒ-, -(polypropylene oxide)ₙ(polyethylene oxide)ₒ-, where n and o independently of one another denote numbers from 0 to 200, and the distribution of the EO/PO units can be random or in the form of blocks, - ((C₁-C₁₀) alkyl) - (Si (OCH₃)₂) - and - (Si (OCH₃)₂) -;
R³, R⁴, R⁵, and R⁶ independently of one another are -CH₃, -O-CH₃, -C₆H₅ or -O-C₆H₅;
w, x denote numbers from 0 to 500, it being necessary for either w or x to be greater than zero, and
R² is a saturated or unsaturated aliphatic, cycloaliphatic, arylaliphatic or aromatic radical having in each case 1 to 50 carbon atoms or a group of the formulae -OH, -NH₂, -N(CH₃)₂ , -R⁷ or a group -Z-R¹, where Z and R¹ have the meanings mentioned above, and
R⁷ is a group of the formula -O-Si(CH₃)₃, -O-Si(phenyl)₃, -O-Si (O-Si (CH₃)₃)₂CH₃) and -O-Si(O-Si(Ph)₃)₂Ph).

5. Use according to at least one of Claims 1 to 4, **characterized in that** the fluorine-containing components E) are compounds of the formula (II)
R¹-Y-CᵣH₂ᵣCₛF₂ₛCF₃ (II)
where
R¹ represents a polymerizable function from the group of the vinylically unsaturated compounds, preferably a vinyl, allyl, methallyl, methylvinyl, acryloyl, methacryloyl, crotonyl, senecionyl, itaconyl, maleyl, fumaryl or styryl radical;
Y is a chemical bridge, preferably -0-, -C(O)-, -C(O)-O-, -S-, -O-CH₂-CH(O-)-CH₂OH, -O-CH₂-CH(OH)-CH₂-O-, -O-SO₂-O-, -O-S(O)-O-, -PH-, -P(CH₃) -, -PO₃-, -NH-, -N(CH₃)-, -O-(C₁-C₅₀)alkyl-O-, -O-phenyl-O-, -O-benzyl-O-, -O-(C₅-C₈)cycloalkyl-O-, -O-(C₁-C₅₀)alkenyl-O-, -O-(CH(CH₃)-CH₂-O)ₙ-, -O-(-CH₂-CH₂-O)ₙ-, and -O-([CH-CH₂-O]ₙ-[CH₂-CH₂-O]ₘ)ₒ-, where n, m, and o independently of one another denote numbers from 0 to 200, and
r, s are stoichiometric coefficients which independently of one another are numbers between 0 and 200.

6. Use according to at least one of Claims 1 to 5, **characterized in that** the polymeric additives G) are homopolymers or copolymers of N-vinylformamide, N-vinylacetamide, N-vinylpyrrolidone, ethylene oxide, propylene oxide, acryloyldimethyltaurine, N-vinylcaprolactam, N-vinylmethylacetamide, acrylamide, acrylic acid, methacrylic acid, N-vinylmorpholide, hydroxymethyl methacrylate, diallyldimethylammonium chloride (DADMAC) and/or [2-(methacryloyloxy)-ethyl]trimethylammonium chloride (MAPTAC); polyalkylene glycols and/or alkylpolyglycols.

7. Use according to at least one of Claims 1 to 6, **characterized in that** the copolymerization takes place in the presence of at least one polymeric additive G).

8. Use according to at least one of Claims 1 to 7, **characterized in that** the copolymers are crosslinked.

9. Use according to at least one of Claims 1 to 8, **characterized in that** the copolymers are prepared by precipitation polymerization in tert-butanol.

10. Use according to at least one of Claims 1 to 9, **characterized in that**, based on the finished compositions, from 0.01 to 10% by weight of the copolymers are used.

11. Use according to at least one of Claims 1 to 10, **characterized in that** the copolymers are water-soluble or water-swellable.

## Revendications

1. Utilisation de copolymères, pouvant être obtenus par copolymérisation radicalaire de
A) de l'acide acryloyldiméthyltaurique et/ou des taurates d'acryloyldiméthyle,
B) éventuellement un ou plusieurs comonomères supplémentaires oléfiniquement insaturés, non cationiques, éventuellement réticulants, qui comprennent au moins un atome d'oxygène, d'azote, de soufre ou de phosphore et présentent un poids moléculaire inférieur à 500 g/mol,
C) éventuellement un ou plusieurs comonomères cationiques oléfiniquement insaturés, qui comprennent au moins un atome d'oxygène, d'azote, de soufre ou de phosphore, et présentent un poids moléculaire inférieur à 500 g/mol,
D) éventuellement un ou plusieurs composants au moins monofonctionnels, aptes à la polymérisation radicalaire, contenant du silicium,
E) éventuellement un ou plusieurs composants au moins monofonctionnels, aptes à la polymérisation radicalaire, contenant du fluor,
F) un ou plusieurs macromonomères mono- ou polyoléfiniquement insaturés, éventuellement réticulants, qui comprennent à chaque fois au moins un atome d'oxygène, d'azote, de soufre ou de phosphore, et présentent un poids moléculaire moyen en nombre supérieur ou égal à 200 g/mol, les macromonomères n'étant pas un composant D) contenant du silicium ou un composant E) contenant du fluor, et les macromonomères étant des éthoxylates d'alkyle acryliquement ou méthacryliquement monofonctionnalisés de formule (IV) 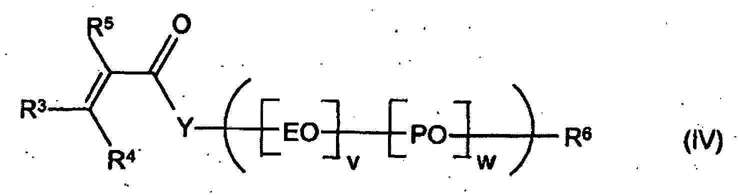 dans laquelle
R₃ et R₄ représentent H ou -CH₃, R₅ représente H ou -CH₃; et R₆ représente un radical hydrocarboné en (C₁-C₃₀) n-aliphatique, iso-aliphatique, oléfinique, cycloaliphatique, arylaliphatique ou aromatique,
v et w sont les coefficients stoechiométriques correspondant aux unités oxyde d'éthylène (EO) et aux unités oxyde de propylène (PO), v et w valant indépendamment l'un de l'autre 0 à 500, la somme de v et w devant en moyenne être ≥ 1, et la distribution des unités EO et PO dans la chaîne macromonomère pouvant être statistique, séquencée, alternée ou en gradient, et Y représente -O-, -C(O)-, -C(O)-O-, -S-, -O-CH₂-CH(O)-CH₂OH, -O-CH₂-CH (OH) -CH₂O-, -O-SO₂-O-, -O-SO-O-, -PH-, -P(CH₃)-,-PO₃-,-NH- ou -N(CH₃)-,
G) la copolymérisation ayant éventuellement lieu en présence d'au moins un additif polymère ayant des poids moléculaires moyens en nombre de 200 g/mol à 10⁹ g/mol, en tant qu'épaississant, dispersant, agent de suspension, émulsifiant, stabilisateur, solubilisant, conditionneur, donneur de consistance, lubrifiant, promoteur d'adhésion et/ou conditionneur dans des agents cosmétiques, pharmaceutiques et dermatologiques.

2. Utilisation selon la revendication 1, **caractérisée en ce que** les comonomères B) sont des acides carboxyliques insaturés, des sels d'acides carboxyliques insaturés, des anhydrides d'acides carboxyliques insaturés, des esters d'acides carboxyliques insaturés avec des alcools aliphatiques, oléfiniques, cycloaliphatiques, arylaliphatiques ou aromatiques contenant 1 à 22 atomes C, des N-vinylamides à chaîne ouverte, des N-vinylamides cycliques d'une taille de cycle de 3 à 9, des amides de l'acide acrylique, des amides de l'acide méthacrylique, des amides d'acides acryliques substitués, des amides d'acides méthacryliques substitués, la 2-vinylpyridine, la 4-vinylpyridine, l'acétate de vinyle ; le styrène, l'acrylonitrile, le chlorure de vinyle, le chlorure de vinylidène, le tétrafluoroéthylène, l'acide vinylphosphonique ou ses esters ou sels, l'acide vinylsulfonique ou ses esters ou sels, l'acide allylphosphonique ou ses esters ou sels et/ou l'acide méthallylsulfonique ou ses esters ou sels.

3. Utilisation selon la revendication 1 et/ou 2, **caractérisée en ce que** les comonomères C) sont le chlorure de diallyldiméthylammonium (DADMAC), le chlorure de [2-(méthacryloyloxy)éthyl]triméthylammonium (MAPTAC), le chlorure de [2-(acryloyloxy)éthyl]triméthylammonium, le chlorure de [2-méthacrylamidoéthyl]triméthylammonium, le chlorure de [2-(acrylamido)éthyl]triméthylammonium, le chlorure de N-méthyl-2-vinylpyridinium, le chlorure de N-méthyl-4-vinylpyridinium, le méthacrylate de diméthylaminoéthyle, le diméthylaminopropylméthacrylamide, le N-oxyde de méthacryloyléthyle et/ou la méthacryloyléthyl-bétaïne.

4. Utilisation selon au moins l'une quelconque des revendications 1 à 3, **caractérisée en ce que** les composants D) contenant du silicium sont des composés de formule (I)
R1-Z-[(Si(R³R⁴)-O-)_{w}-(Si(R⁵R⁶)-O)ₓ-] -R² (I)
dans laquelle
R¹ représente un radical vinyle, allyle, méthallyle, méthylvinyle, acryle, méthacryle, crotonyle, sénécionyle, itaconyle, maléinyle, fumaryle ou styryle ;
Z représente un pont chimique, de préférence choisi parmi -O-, -(alkylène en (C₁-C₅₀)-, -(arylène en (C₆-C₃₀))-, -(cycloalkylène en (C₅-C₈)-, -(alcénylène en (C₁-C₅₀)-, -(oxyde de polypropylène)ₙ-, -(oxyde de polyéthylène)ₒ-, -(oxyde de polypropylène)ₙ(oxyde de polyéthylène)ₒ-, n et o signifiant indépendamment l'un de l'autre des nombres de 0 à 200 et la distribution des unités EO/PO pouvant être statistique ou séquencée, -(alkyle en (C₁-C₁₀)-(Si(OCH₃)₂) - et -(Si(OCH₃)₂)- ;
R³, R⁴, R⁵ et R⁶ signifient indépendamment les uns des autres -CH₃, -O-CH₃, -C₆H₅ ou -O-C₆H₅ ;
w, x signifient des nombres de 0 à 500, w ou x devant être supérieur à zéro, et
R² signifie un radical saturé ou insaturé, aliphatique, cycloaliphatique, arylaliphatique ou aromatique, contenant à chaque fois 1 à 50 atomes C, ou un groupe de formule -OH, -NH₂, -N(CH₃)₂, -R⁷ ou un groupe -Z-R¹, Z et R¹ ayant les significations susmentionnées, et
R⁷ signifie un groupe de formule -O-Si(CH₃)₃, -0-Si(phényl)₃, -O-Si(O-Si(CH₃)₃)₂CH₃) et -O-Si(O-Si(Ph)₃)₂Ph).

5. Utilisation selon au moins l'une quelconque des revendications 1 à 4, **caractérisée en ce que** les composants E) contenant du fluor sont des composés de formule (II)
R¹-Y-C-ᵣH₂rCₛF₂ₛCF₃ (II)
dans laquelle
R¹ représente une fonction polymérisable du groupe des composés vinyliquement insaturés, de préférence un radical vinyle, allyle, méthallyle, méthylvinyle, acryle, méthacryle, crotonyle, sénécionyle, itaconyle, maléinyle, fumaryle ou styryle ;
Y représente un pont chimique, de préférence -O-, -C(O)-, -C(O)-O-, -S-, -O-CH₂-CH (O-) -CH₂OH, -O-CH₂-CH(OH)-CH₂-O-, -O-SO₂-O-, -O-S(O)-O-, -PH-, -P(CH₃)-, -PO₃-, -NH-, -N(CH₃)-, -O-alkyle en (C₁-C₅₀)-O-, -O-phényl-O-, -O-benzyl-O-, -O-cycloalkyle en (C₅-C₈)-O-, -O-alcényle en (C₁-C₅₀)-O-, -O-(CH(CH₃-CH₂-O)ₙ-, -O-(CH₂-CH₂-O)ₙ- et -O-([CH-CH₂-O]ₙ-[CH₂-CH₂-O]ₘ)ₒ-, n, m et o signifiant indépendamment les uns des autres des nombres de 0 à 200, et
r, s représentent des coefficients stoechiométriques, qui sont indépendamment l'un de l'autre des nombres compris entre 0 et 200.

6. Utilisation selon au moins l'une quelconque des revendications 1 à 5, **caractérisée en ce que** les additifs polymères G) sont des homo- ou copolymères de N-vinylformamide, N-vinylacétamide, N-vinylpyrrolidone, oxyde d'éthylène, oxyde de propylène, acide acryloyldiméthyltaurique, N-vinylcaprolactame, N-vinylméthylacétamide, acrylamide, acide acrylique, acide méthacrylique, N-vinylmorpholide, méthacrylate d'hydroxyméthyle, chlorure de diallyldiméthylammonium (DADMAC) et/ou chlorure de [2-(méthacryloyloxy)éthyl]triméthylammonium (MAPTAC) ; des polyalkylène glycols et/ou des alkylpolyglycols.

7. Utilisation selon au moins l'une quelconque des revendications 1 à 6, **caractérisée en ce que** la copolymérisation a lieu en présence d'au moins un additif polymère G).

8. Utilisation selon au moins l'une quelconque des revendications 1 à 7, **caractérisée en ce que** les copolymères sont réticulés.

9. Utilisation selon au moins l'une quelconque des revendications 1 à 8, **caractérisée en ce que** les copolymères sont fabriqués par polymérisation par précipitation dans du tert.-butanol.

10. Utilisation selon au moins l'une quelconque des revendications 1 à 9, **caractérisée en ce que** 0,01 à 10 % en poids des copolymères sont utilisés par rapport aux agents finis.

11. Utilisation selon au moins l'une quelconque des revendications 1 à 10, **caractérisée en ce que** les copolymères sont solubles dans l'eau ou gonflables dans l'eau.
